(19) **Europäisches Patentamt / European Patent Office / Office européen des brevets**

(11) **EP 4 471 147 A1**

(12) **EUROPEAN PATENT APPLICATION**
published in accordance with Art. 153(4) EPC

(43) Date of publication:
04.12.2024 Bulletin 2024/49

(21) Application number: 23747069.5

(22) Date of filing: 27.01.2023

(51) International Patent Classification (IPC):
*C12N 15/13* (2006.01)    *A61K 39/395* (2006.01)
*A61P 11/00* (2006.01)    *A61P 31/14* (2006.01)
*C07K 16/10* (2006.01)    *C12N 1/15* (2006.01)
*C12N 1/19* (2006.01)    *C12N 1/21* (2006.01)
*C12N 5/10* (2006.01)    *C12N 15/63* (2006.01)
*C12P 21/02* (2006.01)    *C12P 21/08* (2006.01)

(52) Cooperative Patent Classification (CPC):
A61K 39/395; A61P 11/00; A61P 31/14;
C07K 16/00; C07K 16/10; C12N 5/10; C12N 15/63;
C12N 15/74; C12N 15/80; C12N 15/81; C12P 21/02

(86) International application number:
PCT/JP2023/002584

(87) International publication number:
WO 2023/145859 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.01.2022 JP 2022011794
18.03.2022 JP 2022043518
17.10.2022 JP 2022166041

(71) Applicants:
• Evec Inc.
Sapporo-shi, Hokkaido 060-0001 (JP)
• National Center for Global Health and Medicine
Tokyo 162-8655 (JP)
• National Institutes of Biomedical Innovation,
Health and Nutrition
Ibaraki-shi, Osaka 567-0085 (JP)

(72) Inventors:
• MIURA, Ryu
Sapporo-shi, Hokkaido 004-0015 (JP)

• ISHIZAKA, Yukihito
Tokyo 162-8655 (JP)
• UENO, Mikako
Tokyo 162-8655 (JP)
• KUTSUNA, Satoshi
Tokyo 162-8655 (JP)
• SAITO, Sho
Tokyo 162-8655 (JP)
• KIMURA, Moto
Tokyo 162-8655 (JP)
• YASUTOMI, Yasuhiro
Tsukuba-shi, Ibaraki 305-0843 (JP)
• URANO, Emiko
Tsukuba-shi, Ibaraki 305-0843 (JP)

(74) Representative: Müller-Boré & Partner
Patentanwälte PartG mbB
Friedenheimer Brücke 21
80639 München (DE)

Remarks:
The complete document including Reference
Table(s) and the Sequence Listing(s) can be
downloaded from the EPO website

(54) **HUMAN NEUTRALIZING ANTIBODY AGAINST SARS-COV-2 HAVING BREADTH TO VARIANT STRAINS, AND ANTIGEN-BINDING FRAGMENT THEREOF**

(57) In order to provide a novel human-derived monoclonal antibody that binds to a spike (S) protein of a severe acute respiratory syndrome coronavirus 2 (SARS-CoV-2) to neutralize SARS-CoV-2, an antigen-binding fragment thereof, and a pharmaceutical composition containing the antibody or the antigen-binding fragment thereof, the present invention provides: a human-derived monoclonal antibody against SARS-CoV-2, the antibody binding to the S protein of SARS-CoV-2 to neutralize viral activity of the S protein including an immune escape mutation; an antigen-binding fragment thereof; nucleic acids encoding the antibody and the

   **(Cont. next page)**

antigen-binding fragment thereof; and a pharmaceutical composition for treating or preventing a SARS-CoV-2 coronavirus infectious disease, the pharmaceutical composition containing the antibody or the antigen-binding fragment thereof.

[FIG. 4]

**Description**

Technical Field

The present invention generally relates to a monoclonal antibody that binds to a spike (S) protein of a severe acute respiratory syndrome coronavirus 2 (hereinafter, referred to as "SARS-CoV-2") to neutralize biological activity of SARS-CoV-2, an antigen-binding fragment thereof, and use thereof as a medicament.

Background Art

A new coronavirus SARS-CoV-2 that first emerged in Wuhan, China in 2019 caused a worldwide epidemic called "pandemic" as a severe acute respiratory disease novel coronavirus infectious disease (hereinafter, referred to as "COVID-19"). As of October 4, 2021, the World Health Organization (WHO) reported 234,809,103 confirmed cases and 4,800,375 deaths. Clinical features of COVID-19 include fever, dry cough, malaise, and the like, which can cause respiratory failure to lead to death.

SARS-CoV-2 has a form of a typical beta coronavirus. Similarly to other coronaviruses, a spherical virus particle having a diameter of about 100 nm is wrapped with an envelope (outer membrane) of a lipid bilayer membrane has a crown-like projection (spike) on a surface of the envelope.

The spike is formed of a spike (S) protein trimer and binds to a human cell membrane angiotensin converting enzyme2 (ACE2) receptor (Non Patent Literatures 1 and 2). After binding, the spike is activated by a host cell protease (Non Patent Literatures 3 and 4), the viral envelope and a host cell membrane fuse, and the virus enters a cytoplasm. That is, infection can be suppressed by inhibiting binding between the spike and ACE2, which is an initial stage for the virus to infect a cell, and therefore the S protein is a target of antibody therapy including a vaccine (Non Patent Literature 5).

SARS-CoV-2 has a positive single-stranded RNA containing about 30,000 bases as a genome. It is known that RNA is less stable than DNA and is prone to spontaneous mutation. Also in SARS-CoV-2, generation of a variant has been reported from various countries and regions. The variant has characteristics such as an increase in infectivity, a change in antigenicity, and a decrease in vaccine efficacy due to immune deviation depending on a mutation site thereof. WHO designates a variant of concern (VOC) and a variant of interest (VOI) depending on severity of a mutation of the variant, and is observing epidemic situations of these variants.

As of October 4, 2021, an alpha strain (B.1.1.7: referred to as "English type"), a beta strain (B. 1.351: referred to as "South African type"), a gamma strain (P. 1: referred to as "Brazilian type"), and a delta strain (B.1.617.2: referred to as "Indian type") with mutations that are believed to affect infectivity and transmission were designated as VOC. In addition to these four variants, WHO named a new variant detected in South Africa and the like an omicron strain (B.1.1.529) on November 26, 2021, and designated the omicron strain as VOC.

"Vaccine development" progressed at a surprising speed, and infection convergence of the alpha strain as a mainstream was successful through vaccination mainly in Europe and America from the end of 2020. However, the mainstream of infection was replaced with the delta strain, and about 88% of newly infected people were infected by the delta strain. A characteristic of the delta strain is a mutation called "L452R". This indicates that the 452nd amino acid of an S protein used when the virus enters a cell has been mutated from L (leucine) to R (arginine). According to a report by Center for Disease Control (CDC), it was confirmed that the delta strain was more than twice as contagious as the conventional novel coronavirus.

According to analysis by Center for Surveillance, Immunization, and Epidemiologic Research of the National Institute of Infectious Diseases as of August 23, 2021, even in Japan, a ratio of cases with delta strain mutation among SARS-CoV-2 positive cases was estimated to be 99% in the Kanto region and 96% in the Kansai region, and it was in a situation of facing a "fifth wave" in which the mainstream of infection was almost replaced with the delta strain. However, infection tended to converge after September 2021, and an expert organization advising the Ministry of Health, Labour and Welfare evaluated an infection situation across the whole country as "decreasing to the level before the fifth wave infection spread" at the meeting on October 6.

This rapid infection convergence seems to be caused by vaccination and the like, but a reason thereof is not clear. Meanwhile, for example, in the United Kingdom at the time when about 60% of the total population finished vaccination, the number of newly infected people stopped decreasing, and there was no sign of convergence. The largest problem in emergence of the delta strain is that a threshold for achieving population immunity, which was regarded as "60% to 70%" in the alpha strain, rose to "85%". In addition, it is pointed out that there may a difference between individuals in effect of a vaccine, and some people have health problems that they cannot be vaccinated. As a matter of fact, there is also a feeling of "vaccine repellency". It is not possible to acquire population immunity worldwide, and the novel coronavirus may continue to spread and proliferate anywhere in the world.

In fact, new variants have emerged. A lambda strain that was first reported in Peru in August 2020 and a mu strain that was first reported in Columbia in January 2021 were designated as VOI on September 30. Infection with each of these strains spread mainly in South American countries. An omicron strain that was reported in South Africa on November 24, 2021 is more infectious than other variants, and is now raging in countries of the world including Japan. As subordinate lineages of the omicron strain, a BA. 1 lineage, a BA.2 lineage, a BA.3 lineage, a BA.4 lineage, and a BA.5 lineage are designated. In Japan, around February 2022, the delta strain was replaced with the BA. 1 lineage of the omicron strain and then replaced with the BA.2 lineage in the whole country, and then replaced with the BA.5 lineage and its sublineage, and the situation had entered the eighth wave by the end of 2022. In addition, various recombinants between the omicron strains have also been reported in Japan and abroad. There is a high possibility that virus mutation will continuously occur due to local infection spread in the future.

As described above, in view of the current situation where the infection cannot be completely suppressed only by a vaccine, it is essential to develop a therapeutic agent that can cope with a variant. Although development of a small-molecule oral drug as a therapeutic agent has progressed, there are current concerns about a risk of emergence of a new drug-resistant strain in each drug, action on a protein other than a target (problem of specificity), long-term safety, and the like. Meanwhile, a neutralizing antibody has superiority in terms of its specificity, the length of a blood half-life, a long-term administration effect, and the like, and has already exhibited an effect in a clinical field as a therapeutic agent. However, the largest risk for a future infection situation is "immune escape mutation" that has acquired ability to escape from the neutralizing antibody.

Citation List

Non Patent Literature

Non Patent Literature 1: Wu F. et al. Nature, 2020; Mar; 579 (7798): 265-269
Non Patent Literature 2: Zhou P. et al. Nature, 2020; Mar; 579 (7798): 270-273
Non Patent Literature 3: Hoffmann M. et al. Cell, 2020 Apr16; 181(2): 271-280. e8
Non Patent Literature 4: Walls AC. et al. Cell, 2020 Apr16; 181(2): 281-292. e6
Non Patent Literature 5: Wu Y. et al. Science, 2020 June 12; 368 (6496): 1274-1278

Summary of Invention

Technical Problem

Under such a circumstance, in order to cope with a variant including immune escape mutation, there is a demand for development of a novel antibody drug for treating or preventing a SARS-CoV-2 infectious disease, the antibody drug containing a monoclonal antibody having a wide neutralizing activity spectrum against a SARS-CoV-2 variant or an antigen-binding fragment thereof.

Solution to Problem

For the purpose of developing a novel antibody drug having broad specificity to a SARS-CoV-2 variant, the present inventors attempted to acquire an antibody from a B lymphocyte separated from peripheral blood of a person who has recovered from wild form SARS-CoV-2 infection. As a result, the present inventors have found a human-derived monoclonal antibody that binds to a SARS-CoV-2 S protein to neutralize viral activity of a plurality of variants including all the five variants of concern (VOC). In addition, the antibody also exhibited an inhibitory effect on immune escape variants of a cocktail antibody (mAb10933 and mAb 10987 (hereinafter, referred to as "Reg10933+Reg10987")) from Regeneron (WO 2021/045836 A1) and Bebtelovimab (LY-CoV-1404) from Eli Lilly which was the only one agent that exhibited activity on strains from a wild strain to omicron BA.5 among approved antibody therapeutic agents (US 11447541 B1).

Therefore, the present disclosure includes the following [1] to [15].

[1] A monoclonal antibody against SARS-CoV-2 coronavirus and an antigen-binding fragment thereof, wherein the monoclonal antibody binds to an S protein of SARS-CoV-2 coronavirus to neutralize viral activity.
[2] The antibody or the antigen-binding fragment thereof according to [1], including:

a heavy chain variable region including an amino acid sequence of SEQ ID NO: 1, SEQ ID NO: 11, or SEQ ID NO: 20, or an amino acid sequence having 90% or more of identity to the amino acid sequence of SEQ ID NO: 1, SEQ ID NO: 11, or SEQ ID NO: 20; and
a light chain variable region including an amino acid sequence of SEQ ID NO: 5 or SEQ ID NO: 21, or an amino acid sequence having 90% or more of identity to the amino acid sequence of SEQ ID NO: 5 or SEQ ID NO: 21.

[3] The antibody or the antigen-binding fragment thereof according to [1] or [2], including:

a heavy chain including an amino acid sequence of SEQ ID NO: 9, SEQ ID NO: 12, or SEQ ID NO: 17, or an amino acid sequence having 90% or more of identity to the amino acid sequence of SEQ ID NO: 9, SEQ ID NO: 12, or SEQ ID NO: 17; and
a light chain including an amino acid sequence of SEQ ID NO: 10 or SEQ ID NO: 19, or an amino acid sequence having 90% or more of identity to the amino acid sequence of SEQ ID NO: 10 or SEQ ID NO: 19.

[4] The antibody or the antigen-binding fragment thereof according to any one of [1] to [3], wherein

(i) a heavy chain variable region includes:

(a) an amino acid sequence of a heavy chain CDR1 including an amino acid sequence of SEQ ID NO: 2,
(b) an amino acid sequence of a heavy chain CDR2 including an amino acid sequence of SEQ ID NO: 3, and
(c) an amino acid sequence of a heavy chain CDR3 including an amino acid sequence of SEQ ID NO: 4, and

(ii) a light chain variable region includes:

(a) an amino acid sequence of a light chain CDR1 including an amino acid sequence of SEQ ID NO: 6,
(b) an amino acid sequence of a light chain CDR2 including an amino acid sequence of SEQ ID NO: 7, and
(c) an amino acid sequence of a light chain CDR3 including an amino acid sequence of SEQ ID NO: 8.

[5] The antibody or the antigen-binding fragment thereof according to any one of [1] to [4], wherein neutralizing activity (IC50) against either or both of a wild strain and a variant including an alpha strain, a beta strain, a gamma strain, a delta type, and a kappa strain of SARS-CoV-2 is about 100 ng/mL or less, and/or neutralizing activity against an omicron strain of SARS-CoV-2 is about 3 $\mu$g/mL or less.

[6] The antibody or the antigen-binding fragment thereof according to any one of [1] to [5], which also exhibits an inhibitory effect (or has neutralizing activity) on escape variants of a cocktail antibody containing antibodies specified by each of mAb10933 and mAb10987 in WO 2021/045836 or an equivalent thereof and Bebtelovimab (LY-CoV-1404) specified by 1404 in US 11447541 B1.

[7] The antibody or the antigen-binding fragment thereof according to any one of [1] to [6], wherein a $K_D$ value for a receptor binding domain (RBD) of each of a wild strain, an alpha strain, a beta strain, a gamma strain, a delta strain, and a kappa strain (B.1.617. 1: referred to as "Indian type") is about $1.2 \times 10^{-9}$ M or less, and/or a $K_D$ value for RBD of an omicron strain is about $3.3 \times 10^{-8}$ M or less.

[8] The antibody or the antigen-binding fragment thereof according to any one of [1] to [7], containing

(i) an antibody that suppresses SARS-CoV-2 coronavirus proliferation in a primate model, or an antigen-binding fragment thereof, or
(ii) a heavy chain consisting of an amino acid sequence of SEQ ID NO: 9, SEQ ID NO: 12, or SEQ ID NO: 17 and a light chain consisting of an amino acid sequence of SEQ ID NO: 10 or SEQ ID NO: 19.

[9] A pharmaceutical composition for treating or preventing a SARS-CoV-2 coronavirus infectious disease, containing the antibody or the antigen-binding fragment thereof according to any one of [1] to [8] and a pharmaceutically acceptable carrier.
[10]

(1) Use of the antibody or the antigen-binding fragment thereof according to any one of [1] to [8] in treatment or prevention of a SARS-CoV-2 coronavirus infectious disease,
(2) use of the antibody or the antigen-binding fragment thereof according to any one of [1] to [8] in production of a medicament for treating or preventing a SARS-CoV-2 coronavirus infectious disease, or
(3) a method for treating or preventing a SARS-CoV-2 coronavirus infectious disease, including administering the antibody or the antigen-binding fragment thereof according to any one of [1] to [8] to a subject or a patient in need thereof.

[11] The pharmaceutical composition according to [9] or the use or method according to [10], wherein the SARS-CoV-2 is a variant.

[12] The pharmaceutical composition, use, or method according to [11], wherein the variant is an alpha strain, a beta strain, a gamma strain, a delta strain, a kappa strain, or an omicron strain.

[13] An isolated nucleic acid molecule encoding an amino acid sequence of the antibody or the antigen-binding fragment thereof according to any one of [1] to [8], or an isolated nucleic acid molecule that hybridizes with any of these nucleic acid molecules under highly stringent conditions.

[14] A recombinant expression vector into which the isolated nucleic acid molecule according to [13] is incorporated.

[15] An isolated host cell into which the recombinant expression vector according to [14] is introduced.

[16] A method for producing the antibody or the antigen-binding fragment thereof according to any one of [1] to [8], including culturing the host cell according to [15] and purifying an antibody from the cultured host cell.

Advantageous Effects of Invention

The monoclonal antibody that neutralizes biological activity of SARS-CoV-2 according to the present invention or an antigen-binding fragment thereof binds to an S protein of SARS-CoV-2 of a wild strain or a variant, and loses (neutralizes) biological activity thereof, and thus is useful in prevention or treatment of a SARS-CoV-2 infectious disease. The fully human monoclonal antibody or antigen-binding fragment thereof against an S protein of SARS-CoV-2 according to the present invention has an advantage of having fewer side effects when administered to a human subject or a patient because the fully human monoclonal antibody or the antigen-binding fragment thereof has no immunogenicity to human or has at least reduced immunogenicity to human as compared with an antibody which is not a fully human antibody, and exhibits no immune reaction or exhibits at least reduced immune reaction as compared with an antibody which is not a fully human antibody.

Brief Description of Drawings

Fig. 1 is a flowchart illustrating a production procedure of an anti-SARS-CoV-2 antibody according to the present invention.

Fig. 2 is a diagram illustrating nucleotide sequences of genes encoding a heavy chain and a light chain of an anti-SARS-CoV-2 antibody (EV053286 109S or 109W) according to the present invention and corresponding amino acid sequences. In the drawing, an amino acid is represented by one letter. A light shaded amino acid sequence indicates a variable region amino acid sequence, an underlined amino acid sequence indicates a CDR (IMGT), and a thick shaded amino acid residue (and a corresponding nucleotide sequence) indicates a mutation site.

Fig. 2-2 is a continuation of Fig. 2.

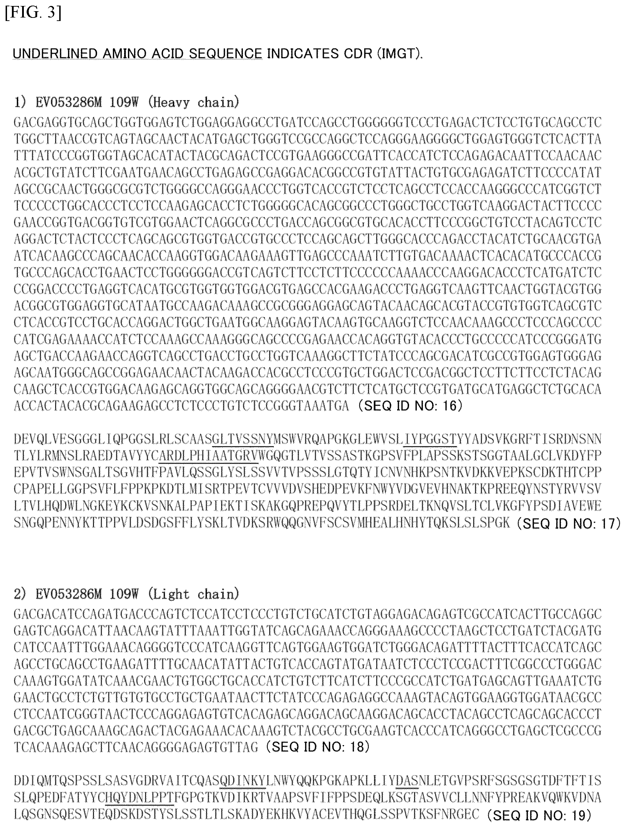

Fig. 3 is a diagram illustrating nucleotide sequences of genes encoding a heavy chain and a light chain of an anti-SARS-CoV-2 antibody (EV053286M 109W) according to the present invention and corresponding amino acid sequences. In the drawing, an amino acid is represented by one letter. An underlined amino acid sequence indicate a CDR (IMGT).

Fig. 4 is a diagram illustrating results of analyzing binding activity of an anti-SARS-CoV-2 antibody (EV053286 109W or EV053286 109S) to a receptor binding site (RBD) of each of a spike protein trimer, a wild strain, an alpha strain, a beta strain, a gamma strain, a delta strain, and a kappa strain by an ELISA method.

Fig. 5 is a diagram illustrating results of analyzing affinity of an anti-SARS-CoV-2 antibody (EV053286 109S) for a receptor binding site (RBD) of each of a wild strain, an alpha strain, a beta strain, a gamma strain, a delta strain, a kappa strain, and an omicron BA. 1 strain using Biacore T-200.

Fig. 6 is a diagram illustrating results of measuring neutralizing activity of an anti-SARS-CoV-2 antibody (EV053286 109S or EV053286 109W) against each of a wild strain, an alpha strain, a beta strain, a gamma strain, a delta strain, a kappa strain, and an omicron BA. 1 strain.

Fig. 7 is a diagram illustrating results of measuring neutralizing activity of each of an anti-SARS-CoV-2 antibody (EV053286 109S) and a Regeneron antibody cocktail (Reg10933+Reg10987) against an omicron BA.2 strain.

Fig. 8 is a diagram illustrating results of measuring neutralizing activity of an anti-SARS-CoV-2 antibody (EV053286 109S) against a Regeneron antibody cocktail (Reg10933+Reg10987) escape variant.

Fig. 9 is a diagram illustrating results of measuring neutralizing activity of an anti-SARS-CoV-2 antibody (EV053286M 109W) against a Bebtelovimab (LY-CoV-1404) escape variant.

Fig. 10 is a diagram illustrating a virus proliferation inhibitory effect in a lung of an anti-SARS-CoV-2 antibody (EV053286M 109W) against an omicron BA.2 strain in a cynomolgus monkey test.

Description of Embodiments

Hereinafter, an embodiment of the present invention will be specifically described with reference to the attached drawings, but the embodiment is intended only to facilitate understanding of the principles of the present invention, and the scope of the present invention is not limited to the following embodiment. It will be understood that another embodiment in which elements of the embodiment of the present invention described below are appropriately replaced with equivalents obvious to those skilled in the art on the basis of disclosure of the present specification and common technical knowledge in the art is also included in the scope of the present invention. Each document cited below for description of the present invention is incorporated herein by reference in its entirety.

1. Explanation of terms

Herein, scientific terms and technical terms used in connection with the present invention have meanings commonly understood by those skilled in the art unless otherwise specified. Furthermore, unless a context requires otherwise, a singular term includes a plurality and a plural term includes a singular. Generally, nomenclature used in connection with techniques of cell culture, molecular biology, immunology, microbiology, genetics, protein, and nucleic acid chemistry described herein is well known and commonly used in the art.

1) Severe acute respiratory syndrome coronavirus 2 (SARS-CoV-2)

"Severe acute respiratory syndrome coronavirus 2" (abbreviated as "SARS-CoV-2") is one kind of Coronaviridae and is a virus belonging to the genus Betacoronavirus. A genome of SARS-CoV-2 consists of a single-stranded RNA with 30 kb, and genes thereof have 80% homology with SARS coronavirus (SARS-CoV), a pandemic of which occurred from 2002 to 2003 (Zhou P. et al., Nature, 579: 270-273, 2020 (Non Patent Literature 2)). A coronavirus particle includes three proteins: a spike (S) protein, an envelope (E) protein, and a membrane (M) protein. A crown-like projection (spike), which is a morphological feature, on a surface of the virus is formed by an S protein trimer.

2) SARS-CoV-2 spike (S) protein

"SARS-CoV-2 S protein" is one of glycoproteins present on a surface of the virus, and is constituted by 1181 amino acid residues (GenBank Protein Accession # QHD43416.1). Herein, a strain of SARS-CoV-2 having this S protein specified by GenBank Protein Accession # QHD43416.1 is referred to as "wild strain". The S protein includes two functional domains: "S1" including an N-terminal domain (NTD) and a receptor binding domain (RBD); and "S2" that mediates fusion of the virus and a host cell membrane. The spike first binds to ACE2 of a host cell via RBD. The spike binding to ACE2 is cleaved and activated by two proteases (TMPRSS2 and furin) of the host cell, fuses a viral membrane with a cell membrane, and sends a viral gene into the cell. Thereafter, a structure wrapped with a lipid bilayer membrane is formed in the cell, and a viral gene is replicated (Non Patent Literature 4). Therefore, infection can be suppressed by inhibiting binding between the spike and ACE2, which is an initial stage of infection.

Herein, "variant" is defined as a strain of SARS-CoV-2 having a mutation in an S protein of wild form SARS-CoV-2. Non-limiting examples of the variant include an alpha strain, a beta strain, a gamma strain, a delta strain, a kappa strain, and an omicron strain.

Herein, the term "SARS-CoV-2" includes a wild form thereof and a variant form thereof unless otherwise specified.

Herein, the terms "immune escape", "immune evasion", "antibody escape", "antibody evasion", and the like are used interchangeably and are used to mean that a host, particularly a human immune system becomes incapable of responding to an infectious pathogen, in other words, a host immune system becomes incapable of recognizing and eliminating a pathogen such as a virus. Herein, particularly, the terms mean that a variant of SARS-CoV-2 coronavirus acquires ability to escape from action of a neutralizing antibody due to a mutation that has occurred in an amino acid sequence of a spike protein of the variant, and a mutation that causes a virus to acquire such antibody escape ability is referred to as "immune escape mutation" or simply "escape mutation", and a variant of a virus having such a mutation is referred to as "immune escape variant" or simply "escape variant".

3) Antibody binding to S protein of SARS-CoV-2

Herein, the term "antibody binding to an S protein of SARS-CoV-2" means an antibody binding to any site of the S protein of SARS-CoV-2, and an epitope may be, for example, a linear epitope, a discontinuous sequence epitope, a conformational epitope, a fragment of the S protein, or the like, and is not limited to RBD. Herein, the terms "anti-SARS-CoV-2 antibody", "antibody capable of neutralizing SARS-CoV-2", "anti-SARS-CoV-2 S protein antibody", "antibody binding to S protein of SARS-CoV-2", "antibody capable of neutralizing biological activity of SARS-CoV-2", and the like are used interchangeably and refer to an antibody that inhibits biological activity of SARS-CoV-2 by binding to the S protein of SARS-CoV-2. As described above, the spike having an important role in an initial stage of infection is formed by an S protein trimer. Note that, in Examples described later, the S protein trimer was used as an antigen. In addition, specific examples of the human monoclonal antibody according to the present invention described in Examples are referred to as "EV053286" herein.

4) Antibody

The term "antibody" as used herein refers to an immunoglobulin molecule formed of four polypeptide chains, that is, two heavy (H) chains and two light (L) chains that are bound to each other by disulfide bonds. The monoclonal antibody in the present invention is also formed of immunoglobulin molecules each containing two heavy chains (H chains) and two light chains (L chains). Each H chain contains an H chain variable part region (also referred to as "HCVR" or "VH") and an H chain invariable part region (The H chain invariable part region contains three domains, which may be referred to as "CH1", "CH2", and "CH3", respectively (collectively referred to as CH). Each L chain contains an L chain variable part region (also referred to as "LCVR" or" VL") and an L chain invariable part region (The L chain invariable part region contains one domain, which may be referred to as "CL".), and a region up to beginning of each invariable part region (also referred to as an invariable region or a constant region) is referred to as a variable part region (or a variable region).

In particular, VH and VL are important in that VH and VL are involved in binding specificity of an antibody. An antibody interacts with a target antigen primarily through amino acid residues in VH and VL. Therefore, an amino acid sequence in the variable part region has a larger difference between individual antibodies than a sequence outside the variable part region. Furthermore, each of VH and VL can also be further subdivided into a region called a framework region (FR) that is kept more constant between various antibodies and a hypervariable region called a complementarity determining region (CDR). Each of VH and VL contains three CDRs and four FRs, which are arranged in order of FR1, CDR1, FR2, CDR2, FR3, CDR3, FR4 from an amino terminal to a carboxy terminal.

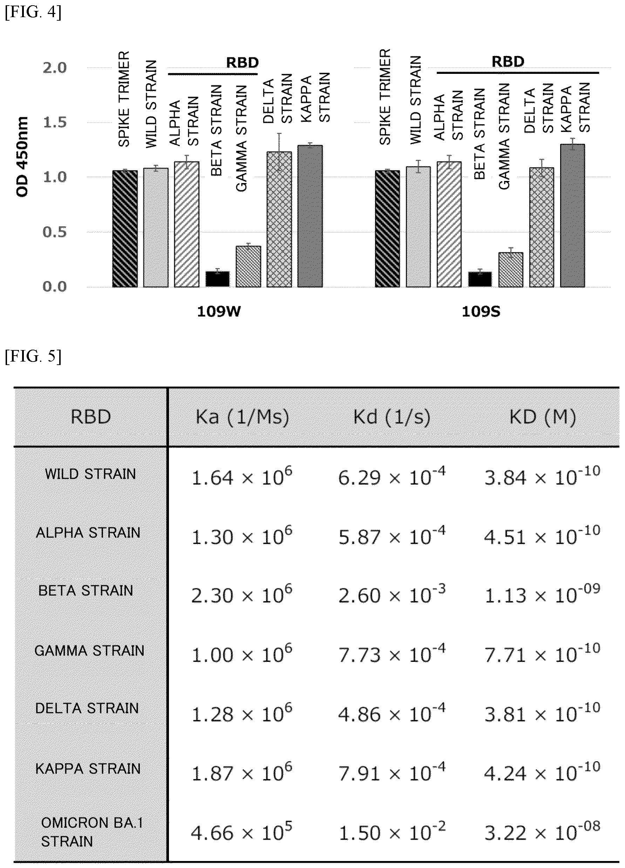

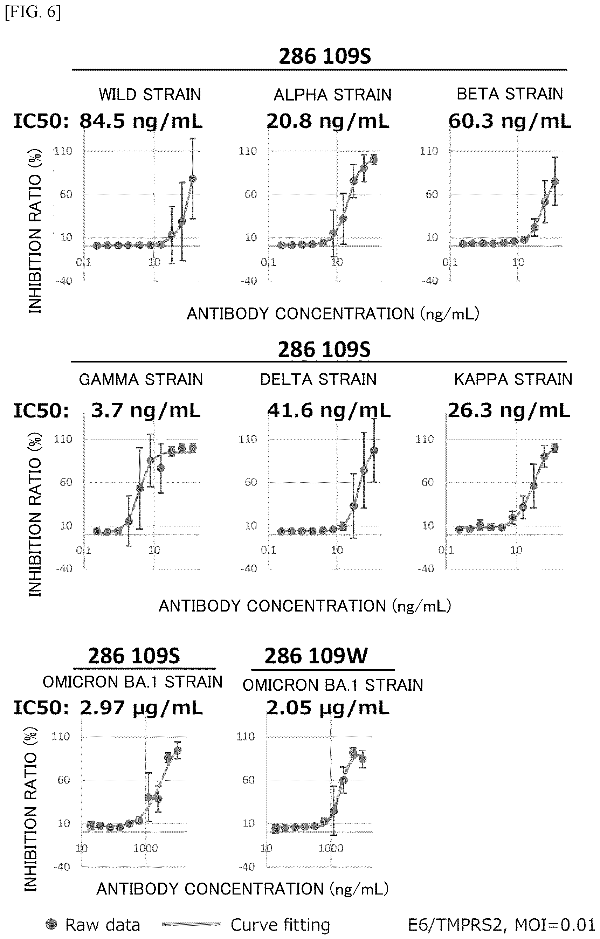

Note that "EV053286" includes a mutation in which the 109th amino acid is tryptophan (109W) or serine (109S), but the mutation is located in FR4, and does not affect activity or does not affect at least an antigen-binding property or infection inhibitory activity of an omicron strain (See "5. Evaluation of antigen-binding property" in Examples, Fig. 4, "7. Evaluation of neutralizing activity" in Examples, and Fig. 6).

On the basis of an amino acid sequence indicating the variable part region and an amino acid sequence of CDR, a human monoclonal antibody and a monoclonal antibody (including a chimeric antibody and a humanized antibody) that can bind to an S protein of SARS-CoV-2 to neutralize a biological activity of SARS-CoV-2, or antigen-binding fragments thereof can be obtained by a well-known technique in the art. These antibodies are within the scope of the present invention.

5) "Antigen-binding fragment" of antibody (or simply "antibody fragment")

The term "antigen-binding fragment" of an antibody (or simply" antibody fragment") as used herein refers to a fragment (for example, VH) of one or more antibodies capable of binding an antigen (S protein of SARS-CoV-2). Note that the fragment also includes a peptide having a minimum amino acid sequence binding to an antigen. Examples of a binding moiety included in the term "antigen-binding fragment" of an antibody include (i) a Fab fragment, (ii) a F(ab')2 fragment, (iii) a Fd fragment containing VH and CH1 domains, (iv) a Fv fragment containing VL and VH domains of a single arm of an antibody, (v) a dAb fragment containing a VH domain (Ward ES. et al., Nature, 341: 544-546, 1989) (vi) an isolated complementarity determining region (CDR) having a framework sufficient for binding, (vii) a bispecific antibody, and (viii) a multispecific antibody. Note that, herein, the term "antibody" without particular distinction includes not only a full-length antibody but also such an "antigen-binding fragment".

6) Isotype

The heavy chain is divided into a $\gamma$ chain, a $\mu$ chain, an $\alpha$ chain, a $\delta$ chain, and an $\epsilon$ chain depending on a difference in constant region, and immunoglobulins of five classes (isotypes) of IgG, IgM, IgA, IgD, and IgE are formed according to this difference, respectively. Furthermore, for humans, IgG has four subclasses of IgG1 to IgG4. Meanwhile, the light chain is divided into a $\kappa$ chain and a $\lambda$ chain depending on a difference in constant region. Note that a class (subclass) of the antibody "EV053286" described in Examples below is IgG1 ($\kappa$).

2. Antibody or antigen-binding fragment thereof according to the present invention

In one aspect, the present invention provides a monoclonal antibody or an antigen-binding fragment thereof (hereinafter, referred to as "antibody of the present invention") that can bind to severe acute respiratory syndrome coronavirus 2 ("SARS-CoV-2") to neutralize "biological activity" (or "viral activity" or" infectivity") of SARS-CoV-2 (These terms are used interchangeably herein.), or to at least suppress infection. Specifically, the antibody of the present invention is an antibody or an antigen-binding fragment thereof capable of binding to an S protein of SARS-CoV-2 in either or both of a wild strain and a variant to neutralize biological activity of the protein. More specifically, the antibody of the present invention is an antibody or an antigen-binding fragment thereof capable of binding to an S protein of SARS-CoV-2 in both of a wild strain and a variant to neutralize biological activity of the protein.

As a method for producing these antibodies, a known method can be used (Riechmann L. et al., Nature, 332: 323-327, 1988). As a non-limiting example of such a method, in the present invention, a method for producing a fully human antibody, including procedures described in Examples below can be used.

A correspondence relationship between a human-derived anti-SARS-CoV-2 antibody and an antigen-binding fragment thereof according to one embodiment of the antibody of the present invention, and amino acid sequence SEQ ID NOs thereof in a sequence listing is presented in Table 1.

[Table 1]

|  | EV053286 109S |  |  | EV053286 109W |
|---|---|---|---|---|
| HCVR | SEQ ID NO: 1 |  | HCVR | SEQ ID NO: 11 |
| HCDR1 | SEQ ID NO: 2 |  | HCDR1 | SEQ ID NO: 2 |
| HCDR2 | SEQ ID NO: 3 |  | HCDR2 | SEQ ID NO: 3 |
| HCDR3 | SEQ ID NO: 4 |  | HCDR3 | SEQ ID NO: 4 |
| LCVR | SEQ ID NO: 5 |  | LCVR | SEQ ID NO: 5 |
| LCDR1 | SEQ ID NO: 6 |  | LCDR1 | SEQ ID NO: 6 |
| LCDR2 | SEQ ID NO: 7 |  | LCDR2 | SEQ ID NO: 7 |
| LCDR3 | SEQ ID NO: 8 |  | LCDR3 | SEQ ID NO: 8 |
| HC | SEQ ID NO: 9 |  | HC | SEQ ID NO: 12 |
| LC | SEQ ID NO: 10 |  | LC | SEQ ID NO: 10 |

A monoclonal antibody EV053286 109S according to one embodiment of the antibody of the present invention includes a heavy chain (HC) consisting of an amino acid sequence of SEQ ID NO: 9 and a light chain (LC) consisting of an amino acid sequence of SEQ ID NO: 10.

In each of the heavy chain and the light chain of EV053286 109S, a heavy chain variable part region consists of an amino acid sequence of SEQ ID NO: 1, and a light chain variable part region consists of an amino acid sequence of SEQ ID NO: 5.

A monoclonal antibody EV053286 109W according to another embodiment of the antibody of the present invention includes a heavy chain (HC) consisting of an amino acid sequence of SEQ ID NO: 12 and a light chain (LC) consisting of an amino acid sequence of SEQ ID NO: 10.

In each of the heavy chain and the light chain of EV053286 109W, a heavy chain variable part region consists of an amino acid sequence of SEQ ID NO: 11, and a light chain variable part region consists of an amino acid sequence of SEQ ID NO: 5.

In the heavy chain variable part region of each of EV053286 109S and EV053286 109W, CDR1 consists of an amino acid sequence of SEQ ID NO: 2, CDR2 consists of an amino acid sequence of SEQ ID NO: 3, and CDR3 consists of an amino acid sequence of SEQ ID NO: 4.

In the light chain variable part region of each of EV053286 109S and EV053286 109W, CDR1 consists of an amino acid sequence of SEQ ID NO: 6, CDR2 consists of an amino acid sequence of SEQ ID NO: 7, and CDR3 consists of an amino acid sequence of SEQ ID NO: 8.

The antibody of the present invention includes not only an antibody including an amino acid sequence such as a specific heavy chain or light chain set forth in SEQ ID NOs: 1 to 12, a variable region thereof, or a CDR thereof, but also an anti-SARS-CoV-2 antibody including an amino acid sequence substantially identical to these amino acid sequences and capable of binding to an S protein of SARS-CoV-2 to neutralize biological activity of SARS-CoV-2. Here, the term "substantially identical" means presence of deletion, substitution, insertion, or addition of one or more amino acid residues in the amino acid sequence of the antibody of the present invention. Alternatively, when any two or more thereof are combined, the term "substantially identical" means presence of deletion, substitution, insertion, or addition of one or more amino acid residues at any position in one or more amino acid sequences in the identical sequence, and two or more of the deletion, substitution, insertion, and addition may occur simultaneously.

Typically, an amino acid sequence that is substantially identical to an amino acid sequence such as a specific heavy chain or light chain set forth in each of SEQ ID NOs: 1 to 12, a variable region thereof, or a CDR thereof contains an amino acid sequence having identity (or sequence identity) of at least, for example, about 80%, 85%, 90%, 91%, 92%, 93%, 94%, 95%, 96%, 97%, 98%, 99%, or more than 99% to each of SEQ ID NOs: 1 to 10.

For example, even a monoclonal antibody containing an amino acid sequence having deletion, substitution, insertion, or addition of amino acid residues of about 20% or less (or about 15% or less, 10% or less, 9% or less, 8% or less, 7% or less, 6% or less, 5% or less, 4% or less, 3% or less, 2% or less, or 1% or less) of the number of amino acid residues in the amino acid sequence of EV053286 described above, or mutation of any two or more of these may be included in the scope of the antibody of the present invention as long as the monoclonal antibody retains activity equivalent to activity (that is, binding to an S protein of SARS-CoV-2 to neutralize viral activity of either or both of a SARS-CoV-2 wild strain and a SARS-CoV-2 variant) of the non-limiting monoclonal antibody (EV053286) described above. One specific example thereof is EV053286M 109W in which one amino acid (D: aspartic acid) is added to an N-terminal of EV053286 109W. Table 2 presents a correspondence relationship between an amino acid sequence in each region of EV053286M 109W and SEQ ID NO.

[Table 2]

|  | EV053286M 109W |
| --- | --- |
| HCVR | SEQ ID NO: 20 |
| HCDCR1 | SEQ ID NO: 2 |
| HCDR2 | SEQ ID NO: 3 |
| HCDR3 | SEQ ID NO: 4 |
| LCVR | SEQ ID NO: 21 |
| LCDR1 | SEQ ID NO: 6 |
| LCDR2 | SEQ ID NO: 7 |
| LCDR3 | SEQ ID NO: 8 |
| HC | SEQ ID NO: 17 |
| LC | SEQ ID NO: 19 |

Therefore, the monoclonal antibody EV053286M 109W according to still another embodiment of the antibody of the present invention contains a heavy chain (HC) consisting of an amino acid sequence of SEQ ID NO: 17 and a light chain (LC) consisting of an amino acid sequence of SEQ ID NO: 19.

In each of the heavy chain and the light chain of EV053286M 109W, a heavy chain variable part region consists of an amino acid sequence of SEQ ID NO: 20, and a light chain variable part region consists of an amino acid sequence of SEQ ID NO: 21.

In the heavy chain variable part region of EV053286M 109W, CDR1 consists of an amino acid sequence of SEQ ID NO: 2, CDR2 consists of an amino acid sequence of SEQ ID NO: 3, and CDR3 consists of an amino acid sequence of SEQ ID NO: 4.

In the light chain variable part region of EV053286M 109W, CDR1 consists of an amino acid sequence of SEQ ID NO: 6, CDR2 consists of an amino acid sequence of SEQ ID NO: 7, and CDR3 consists of an amino acid sequence of SEQ ID NO: 8.

The antibody of the present invention can include an antibody in which an amino acid sequence other than CDR is also derived from human, and can also include a so-called CDR-grafted antibody in which an amino acid sequence other than CDR is derived from another antibody, particularly from another type of antibody. As necessary, a framework region (FR) may have deletion, substitution, insertion, or addition of one to several (specifically, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2, or 1) amino acid residue, or mutation of any two or more of these.

Amino acids constituting a protein in nature can be classified into groups according to characteristics of side chains of the amino acids. For example, as an amino acid group having similar biochemical characteristics, the amino acids can be classified into a group of aromatic amino acids (tyrosine, phenylalanine, and tryptophan), a group of basic amino acids (lysine, arginine, and histidine), a group of acidic amino acids (aspartic acid and glutamic acid), a group of neutral amino acids (serine, threonine, asparagine, and glutamine), a group of amino acids having hydrocarbon chains (alanine, valine, leucine, isoleucine, and proline), and a group of others (glycine, methionine, and cysteine). Substitution of amino acid residues between amino acid residues having side chains with similar biochemical characteristics can be performed while biological activity of the original protein is retained.

For example, amino acid residues (including non-natural amino acids) included in the same group in the following grouping may be mutually substitutable (while biological activity of the original protein is retained). Group A: leucine, isoleucine, norleucine, valine, norvaline, alanine, 2-aminobutanoic acid, methionine, o-methylserine, t-butylglycine, t-butylalanine, and cyclohexylalanine; Group B: aspartic acid, glutamic acid, isoaspartic acid, iso-glutamic acid, 2-aminoadipic acid, and 2-aminosuberic acid; Group C: asparagine and glutamine; Group D: lysine, arginine, omithine, 2,4-diaminobutanoic acid, and 2,3-diaminopropionic acid; Group E: proline, 3-hydroxyproline, and 4-hydroxyproline; Group F: serine, threonine, and homoserine; Group G: phenylalanine, tyrosine, and tryptophan.

Note that identity between amino acid sequences and identity between base sequences can be determined using algorithm BLAST (Karlin S and Altschul SF, PNAS, 87:2264-2268, 1990; PNAS, 90:5873-5877, 1993) by Karlin and Altschul. Programs called BLASTN and BLASTX based on the BLAST algorithm have been developed (Altschul SF. et al., J Mol Biol, 215: 403-410, 1990). When a base sequence is analyzed using BLASTN, parameters are set to, for example, score = 100 and word length = 12. In addition, when an amino acid sequence is analyzed using BLASTX, parameters are set to, for example, score = 50 and word length = 3. When BLAST and Gapped BLAST programs are used, default parameters of each of the programs are used. Alternatively, when identity between amino acid sequences of proteins is determined, amino acid sequences of two types of proteins to be compared are put side by side, and the number of portions having the same amino acid residues is counted, whereby the identity can also be determined by "(the number of the same amino acid residues/the number of amino acid residues in protein full length) $\times$ 100 (%)".

Therefore, typically, in one aspect, the present disclosure provides a monoclonal antibody and an antigen-binding fragment thereof characterized by each of configurations of the following configurations [1] to [8].

[1] A monoclonal antibody against SARS-CoV-2 coronavirus and an antigen-binding fragment thereof, wherein the monoclonal antibody binds to an S protein of SARS-CoV-2 coronavirus to neutralize viral activity.

[2] The antibody or the antigen-binding fragment thereof according to [1], including:

a heavy chain variable region including an amino acid sequence of SEQ ID NO: 1, SEQ ID NO: 11, or SEQ ID NO: 20, or an amino acid sequence having at least 80% (or at least 90% or 95%) of identity to the amino acid sequence of SEQ ID NO: 1, SEQ ID NO: 11, or SEQ ID NO: 20; and

a light chain variable region including an amino acid sequence of SEQ ID NO: 5 or SEQ ID NO: 21, or an amino acid sequence having at least 80% (or at least 90% or 95%) of identity to the amino acid sequence of SEQ ID NO: 5 or SEQ ID NO: 21.

[3] The antibody or the antigen-binding fragment thereof according to [1] or [2], including:

a heavy chain including an amino acid sequence of SEQ ID NO: 9, SEQ ID NO: 12, or SEQ ID NO: 17, or an amino acid sequence having at least 80% (or at least 90% or 95%) of identity to the amino acid sequence of SEQ ID NO: 9, SEQ ID NO: 12, or SEQ ID NO: 17; and

a light chain including an amino acid sequence of SEQ ID NO: 10 or SEQ ID NO: 19 or an amino acid sequence having at least 80% (or at least 90% or 95%) of identity to the amino acid sequence of SEQ ID NO: 10 or SEQ ID NO: 19.

[4] The antibody or the antigen-binding fragment thereof according to any one of [1] to [3], wherein

(i) a heavy chain variable region includes:

(a) an amino acid sequence of a heavy chain CDR1 including an amino acid sequence of SEQ ID NO: 2,
(b) an amino acid sequence of a heavy chain CDR2 including an amino acid sequence of SEQ ID NO: 3, and
(c) an amino acid sequence of a heavy chain CDR3 including an amino acid sequence of SEQ ID NO: 4, and

(ii) a light chain variable region includes:

(a) an amino acid sequence of a light chain CDR1 including an amino acid sequence of SEQ ID NO: 6,
(b) an amino acid sequence of a light chain CDR2 including an amino acid sequence of SEQ ID NO: 7, and
(c) an amino acid sequence of a light chain CDR3 including an amino acid sequence of SEQ ID NO: 8.

[5] The antibody or the antigen-binding fragment thereof according to any one of [1] to [4], wherein neutralizing activity (IC50) against either or both of a wild strain and a variant including an alpha strain, a beta strain, a gamma strain, a delta type, and a kappa strain of SARS-CoV-2 is about 100 ng/mL, and/or neutralizing activity against an omicron strain of SARS-CoV-2 is about 3 $\mu$g/mL or less.

[6] The antibody or the antigen-binding fragment thereof according to any one of [1] to [5], which also exhibits an inhibitory effect (or has neutralizing activity) on escape variants of a cocktail antibody containing antibodies specified by each of mAb10933 and mAb10987 in WO 2021/045836 or an equivalent thereof and an antibody (Bebtelovimab (LY-CoV-1404)) specified by 1404 in US 11447541 B1.

[7] The antibody or the antigen-binding fragment thereof according to any one of [1] to [6], wherein a $K_D$ value for RBD of each of a wild strain, an alpha strain, a beta strain, a gamma strain, a delta strain, and a kappa strain is about 1.2 x $10^{-9}$ M or less, and/or a $K_D$ value for RBD of an omicron strain is about 3.3 x $10^{-8}$ M or less.

[8] The antibody or the antigen-binding fragment thereof according to any one of [1] to [7], containing

(i) an antibody or an antigen-binding fragment thereof that suppresses SARS-CoV-2 coronavirus proliferation in a primate model, or

(ii) a heavy chain consisting of an amino acid sequence of SEQ ID NO: 9, SEQ ID NO: 12, or SEQ ID NO: 17 and a light chain consisting of an amino acid sequence of SEQ ID NO: 10 or SEQ ID NO: 19.

3. Nucleic acid encoding antibody of the present invention

In another aspect, the present invention also provides a nucleic acid molecule encoding an amino acid sequence of the antibody of the present invention. Typically, the present invention provides an isolated nucleic acid molecule encoding amino acid sequences of the monoclonal antibody and antigen-binding fragment thereof characterized by each of [1] to [7] described above.

Furthermore, as a non-limiting example, the present invention provides an isolated nucleic acid having high identity to a nucleic acid (molecule) (polynucleotide) encoding an amino acid sequence of an anti-SARS-CoV-2 antibody or an antigen-binding fragment thereof that can bind to an S protein of SARS-CoV-2 to neutralize biological activity of the S protein (that is, typically, the monoclonal antibody and antigen-binding fragment thereof characterized by each of configurations of [1] to [7] described above) (that is, an isolated nucleic acid selected from nucleic acids that hybridize with the nucleic acid under highly stringent conditions). Here, "having high identity" means sequence identity with which hybridizing can be performed to a predetermined nucleic acid sequence under highly stringent conditions, and for example, means having identity of at least about 60%, 70%, 80%, 85%, 90%, 91%, 92%, 93%, 94%, 95%, 96%, 97%, 98%, 99%, or more than 99%. For example, the nucleic acid is DNA or RNA, and typically DNA.

The "highly stringent conditions" are, for example, conditions of 5×SSC, 5 × Denhardt's solution, 0.5% SDS, 50% formamide, and 50°C. (See, for example., J. Sambrook et al., Molecular Cloning, A Laboratory Manual 2nd ed., Cold Spring Harbor Laboratory Press (1989), particularly section 11.45 "Conditions for Hybridization of Oligonucleotide Probes"). Under these conditions, it can be expected that a polynucleotide (for example, DNA) having higher identity can be efficiently obtained as the temperature is raised. Note that a plurality of factors such as temperature, probe concentration, probe length, ionic strength, time, and salt concentration are conceivable as factors affecting stringency of hybridization, and those skilled in the art can achieve similar stringency by appropriately selecting these factors.

Examples of the nucleic acid that hybridizes under the highly stringent conditions include a nucleic acid having identity of 70% or more, 80% or more, 85% or more, 90% or more, 91% or more, 92% or more, 93% or more, 94% or more, 95% or more, 96% or more, 97% or more, 98% or more, or 99% or more to a nucleic acid encoding an amino acid sequence.

Identity of a base sequence can be determined using the above-described identity search algorithm or the like (Karlin S and Altschul SF, PNAS, 87: 2264-2268, 1990; PNAS, 90: 5873-5877, 1993).

4. Vector, host cell, and method for producing antibody according to the present invention

In still another aspect, the present invention relates to a vector into which the nucleic acid molecule is incorporated, a host cell into which the vector is introduced, and a method for producing an antibody using the vector and the host cell.

The antibody of the present invention can also be produced as a recombinant human antibody using a known method (See Boulianne GL et al., Nature, 312: 643-646,1984; Jones PT et al., Nature, 321: 522-525, 1986, and the like). For example, the antibody of the present invention can be produced by culturing a host cell into which the vector according to the present invention is introduced, and purifying a produced antibody from a culture supernatant or the like. More specifically, the antibody of the present invention can be produced by inserting cDNAs encoding VH and VL into animal cell expression vectors containing genes encoding a human antibody CH and/or a human antibody CL produced from the same cell or different human cells, respectively, to construct human antibody expression vectors, and introducing the human antibody expression vectors into animal cells for expression.

The vector incorporating the nucleic acid encoding VH or VL of the antibody of the present invention is not necessarily limited, but a vector that is widely used for expression of a protein gene and the like and is particularly suitable for expression of an antibody gene or a vector for high expression can be used. Non-limiting examples thereof include a vector containing an FE promoter and/or a CMV enhancer. In addition, usually, an expression vector incorporating a nucleic acid encoding VH and an expression vector incorporating a nucleic acid encoding VL are produced and co-transfected into a host cell, but the nucleic acids encoding VH and VL may be incorporated into a single expression vector.

The host cell into which the expression vector is introduced is not necessarily limited, but includes a cell that is widely used for expression of a protein gene and the like and is particularly suitable for expression of an antibody gene. Examples thereof include bacteria (E. coli and the like), actinomycetes, yeast, insect cells (SF9 and the like), and mammalian cells (COS-1, CHO, myeloma cells, and the like).

In order to industrially produce a recombinant antibody, a recombinant animal cell line that stably and highly produces the antibody, for example, a CHO cell line is generally used. Known methods can be used for production, cloning, gene amplification for high expression, and screening of such a recombinant cell line (See, for example, Omasa T., J. Biosci. Bioeng., 94:600-605, 2002).

The recombinant antibody includes, in addition to an antibody containing two heavy chains and two light chains, an antigen-binding fragment such as Fab (fragment of antigen binding), Fab', or $F(ab')_2$, one obtained by binding an active fragment of an antibody with a linker or the like (for example, a single chain antibody (single chain Fv: scFv) or a disulfide stabilized antibody (disulfide stabilized Fv: dsFv)), and a peptide containing an active fragment of an antibody (for example, a peptide containing CDR). These antibodies can be produced by a known method such as a method for treating the antibody of the present invention with an appropriate protease or a gene recombination technique.

Purification of an antibody can be performed using a known purification means such as a salting-out method, a gel filtration method, an ion exchange chromatography method, or an affinity chromatography method.

In addition, it is also possible to obtain a specific antibody by artificially shuffling VH and VL genes and expressing a diversified scFv (single chain fragment of variable region) antibody as a phage fusion protein by a phage display antibody technique in which a recombinant antibody is expressed on a phage surface by using a genetic engineering technique. This technique is highly evaluated as a humanized antibody production technique that is capable of immune evasion and substitutes for a cell fusion method. A specific antibody or an antigen-binding fragment thereof produced with reference to, for example, amino acid sequences of SEQ ID NOs: 2 to 4 and SEQ ID NOs: 6 to 8 herein using this technique is also within the scope of the present invention.

Furthermore, an antibody obtained by applying a Potellegent technique that significantly improves ADCC activity of an antibody by modifying a sugar chain portion of the antibody to the antibody of the present invention (See Niwa R et al., Clin. Cancer Res., 10:6248-6255, 2004) and an antibody obtained by applying a Complegnent technique that improves CDC activity to the antibody of the present invention (See Kanda Y. et al., Glycobiology, 17: 104-118, 2007.) are also within the scope of the present invention. Similarly, an antibody obtained by a method for modifying ADCC activity (WO 2007/039682 and WO 2007/100083) or CDC activity (WO 2007/011041 and WO 2011/091078) by modifying an amino acid sequence of a constant region portion of the antibody is also within the scope of the present invention.

Furthermore, an antibody or an antigen-binding fragment thereof obtained by applying a partial substitution technique of an Fc region performed for adding protease resistance capability to the antibody and enabling oral administration (See WO 2006/071877) is also within the scope of the present invention.

Note that, as a method for producing an antibody, a polyclonal antibody or a monoclonal antibody is usually acquired using a laboratory animal such as a mouse, a rabbit, or a goat. However, since the antibody thus obtained has a sequence characteristic of an animal species used, when the antibody is directly administered to a human, the antibody is recognized as a foreign substance by a human immune system, and a human anti-animal antibody response may occur (That is, an antibody of the antibody may be generated.).

The anti-SARS-CoV-2 monoclonal antibody or antigen-binding fragment thereof according to the present invention can be obtained from antibody-producing cells derived from blood of a person who has recovered from SARS-CoV-2 infection, and is a fully human antibody. This fully human antibody has an advantage that, even when the fully human antibody is administered to a human body as an antibody medicament, the fully human antibody has no immunogenicity or has at least reduced immunogenicity as compared with an antibody that is not a fully human antibody, and no immune reaction is observed or immune reaction is at least reduced as compared with an antibody that is not a fully human antibody.

5. Pharmaceutical composition containing antibody of the present invention, use of the antibody of the present invention as medicament, use of the antibody of the present invention for production of medicament, and method of treatment or prevention including administration of the antibody of the present invention to patient

In still another aspect, the present invention includes use of the antibody of the present invention as a medicament. In one embodiment, there is provided a pharmaceutical composition for preventing or treating a SARS-CoV-2 infection, the pharmaceutical composition containing the antibody of the present invention and a pharmaceutically acceptable carrier. In one embodiment, there is provided use of the antibody of the present invention in treatment or prevention of a SARS-CoV-2 coronavirus infectious disease. Furthermore, in one embodiment, there is provided use of the antibody of the present invention in production of a medicament for treatment or prevention of a SARS-CoV-2 coronavirus infectious disease. Furthermore, in one embodiment, there is provided a method for treating or preventing a SARS-CoV-2 coronavirus infectious disease, the method including administering the antibody of the present invention to a subject or a patient in need thereof.

In particular, the anti-SARS-CoV-2 antibody or antigen-binding fragment thereof according to the present invention binds to an S protein of SARS-CoV-2 and has high neutralizing capacity, and is therefore useful as a prophylactic or therapeutic agent for a disease in which SARS-CoV-2 is involved.

The term "pharmaceutically acceptable carrier" as used herein includes any or all solvents, dispersion media, coatings, isotonic agents, absorption delaying agents, and the like that are physiologically compatible.

Examples of the pharmaceutically acceptable carrier include one or more selected from water, a salt solution, a phosphate buffered saline, dextrose, glycerol, ethanol, and the like, and combinations thereof. When the pharmaceutically acceptable carrier is used as an injection or the like, a pH adjusting agent or an isotonic agent, for example, a sugar, a polyalcohol such as mannitol or sorbitol, or sodium chloride can be contained in a composition. The pharmaceutically acceptable carrier can further contain a small amount of auxiliary substance that increases preservability or effectiveness of an antibody or an antibody moiety, such as a wetting agent, an emulsifying agent, a preservative, a buffering agent, or a stabilizing agent.

The pharmaceutical composition of the present invention can be in various dosage forms. Examples of such a composition include liquid, semi-solid, and solid dosage forms such as a solution (for example, an injectable and infusible solution), a dispersion, a suspension, a tablet, a capsule, a troche, a pill, a powder, a liposome, and a suppository. The dosage form may vary depending on an intended dosage form and an application example of treatment. For example, those in a form of injectable or infusible solution are included, such as a composition similar to those commonly used to passively immunize a human with another antibody. For example, the dosage form may be parenteral (for example, intravenous, subcutaneous, transdermal, intraperitoneal, intramuscular, transpulmonary, or pernasal). Alternatively, the antibody can be administered by intravenous infusion or intravenous injection. Alternatively, the antibody can be administered by intramuscular, subcutaneous, or intradermal injection. Alternatively, the antibody can be administered by inhalation or pernasally.

6. Method for acquiring anti-SARS-CoV-2 antibody and antigen-binding fragment thereof according to the present invention

Next, a method for obtaining the anti-SARS-CoV-2 monoclonal antibody and antigen-binding fragment thereof according to the present invention will be described, but the method for obtaining the antibody and the like according to the present invention is not limited to the description at all, and as described above, change or modification can be made within the scope of ordinary creativity of those skilled in the art.

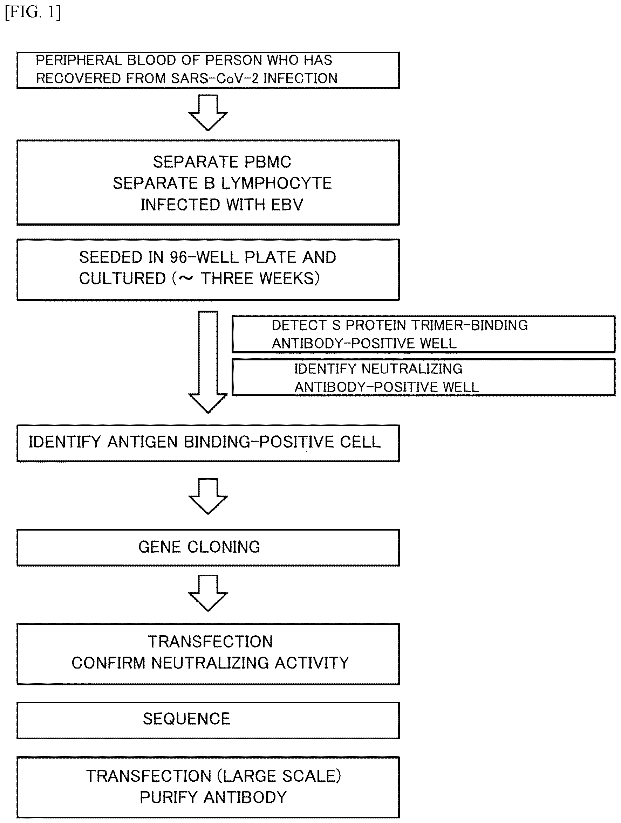

The anti-SARS-CoV-2 antibody and antigen-binding fragment thereof according to the present invention can be obtained by isolating a cell clone that produces the antibody from blood of a person who has recovered from SARS-CoV-2 infection through various steps, cloning a DNA encoding the target antibody, and then performing gene expression and antibody production. Fig. 1 illustrates a flowchart of the above steps of producing the anti-SARS-CoV-2 antibody according to the present invention.

1) Proliferation induction of fully human antibody-producing cells against S protein of SARS-CoV-2

B lymphocytes are separated from blood of a person who has recovered from SARS-CoV-2 infection, and proliferation of the B lymphocytes is induced. A proliferation induction method itself is known, and for example, the proliferation induction can be performed by a transform method (Kozbor D. and Roder JC, Immunol Today, 4:72-9, 1983) using "Epstein-Barr virus (EB virus)" (hereinafter, referred to as "EBV") as an incentive factor for cancer.

2) Isolation of fully human antibody-producing cell clone against S protein of SARS-CoV-2

Antibody-producing cells are identified from the B lymphocyte group on which proliferation induction has been performed by infection with the EBV by ELISA in which an S protein trimer is immobilized and measurement of neutralizing activity of a culture supernatant. In some cases, the B lymphocytes are seeded on a cell microarray, and a single cell that produces an S protein-binding antibody is selected and isolated using a slide glass or microbeads on which an S protein trimer is immobilized.

3) Gene acquisition and antibody production of fully human antibody against S protein of SARS-CoV-2

cDNA is synthesized from the isolated S protein-binding antibody-producing B lymphocytes described above, and the antibody gene is cloned. The acquired gene is introduced into a CHO cell, an antibody is expressed, and an antigen-binding property and neutralizing activity are measured.

The anti-SARS-CoV-2 antibody obtained as described above is a fully human antibody produced from B lymphocytes sensitized in a human body, and therefore has an advantage that a possibility of immune reaction to the antibody when administered to a human is low.

In addition, it is also characterized in that the EB virus having activity of performing proliferation induction by infecting B lymphocytes is used. The EB virus method has an advantage that a native antibody circulating in a human body can be acquired, and an antibody can be acquired even from cells having a very low abundance.

7. Method for evaluating in vitro activity

An antigen-binding property of an antibody or an antibody composition can be evaluated by an ELISA method, a surface plasmon resonance (SPR) method, and the like. Ability of the antibody to suppress biological activity of an S protein of SARS-CoV-2 in vitro can be evaluated by a receptor binding inhibition assay method such as ELISA, a neutralization assay method, and the like.

1) Antigen binding activity

In order to measure a binding property between an antibody and an S protein of SARS-CoV-2, a known method can be used. For example, ELISA using the S protein trimer as an antigen can be adopted. Alternatively, binding affinity for the S protein trimer or RBD can be measured by a protein interaction analyzer such as Biacore T200 (registered trademark). The binding affinity ($K_D$ value) is represented by a ratio ($K_D$ = kd/ka) between kd (dissociation constant) and ka (binding constant). Binding affinity ($K_D$ value) of the human anti-SARS-CoV-2 antibody or antigen-binding fragment thereof according to the present invention for an RBD may be about $2 \times 10^{-9}$ or less, about $1.9 \times 10^{-9}$ or less, about $1.8 \times 10^{-9}$ or less, about $1.7 \times 10^{-9}$ or less, about $1.6 \times 10^{-9}$ or less, about $1.5 \times 10^{-9}$ or less, about $1.4 \times 10^{-9}$ or less, about $1.3 \times 10^{-9}$ or less, about $1.2 \times 10^{-9}$ or less, about $1.1 \times 10^{-9}$ or less, about $1 \times 10^{-9}$ M or less, about $9 \times 10^{-10}$ or less, about $8 \times 10^{-10}$ or less, about $7 \times 10^{-10}$ or less, about $6 \times 10^{-10}$ or less, about $5 \times 10^{-10}$ or less, about $4 \times 10^{-10}$ or less, or about $3 \times 10^{-10}$ or less with respect to a wild strain, an alpha strain, a beta strain, a gamma strain, a delta strain, or a kappa strain, and about $4 \times 10^{-8}$ or less, about $3.9 \times 10^{-8}$ or less, about $3.8 \times 10^{-8}$ or less, about $3.7 \times 10^{-8}$ or less, about $3.6 \times 10^{-8}$ or less, about $3.5 \times 10^{-8}$ or less, about $3.4 \times 10^{-8}$ or less, about $3.3 \times 10^{-8}$ or less, about $3.2 \times 10^{-8}$ or less, about $3.1 \times 10^{-8}$ or less, or about $3 \times 10^{-8}$ M or less with respect to an omicron strain. (See "6. Evaluation of antigen affinity of anti-SARS-CoV-2 antibody" in Examples and Fig. 5)

2) In vitro neutralizing activity

The terms "neutralization", "inhibitory effect", "inhibition", "suppression", "capable of inhibiting", and the like as used herein refer to reducing biological activity resulting from an antigen (SARS-CoV-2) by, for example, at least about 5 to 100%, 10 to 100%, 20 to 100%, 30 to 100%, 40 to 100%, 50 to 100%, 50 to 100%, 70 to 100%, or 80 to 100%.

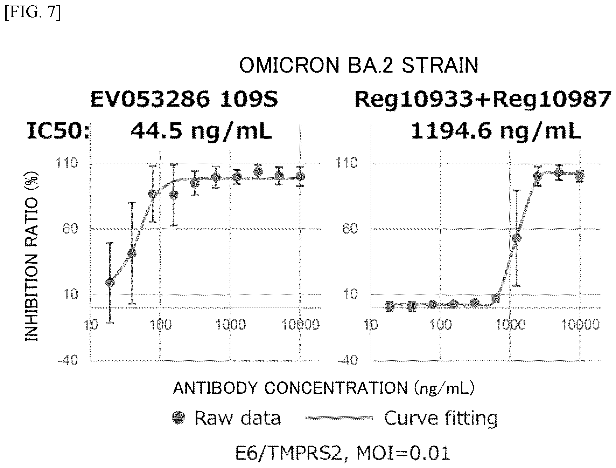

Evaluation of in vitro neutralizing capacity of the anti-SARS-CoV-2 antibody can be performed using a wild strain and/or a variant (Examples: an alpha strain, a beta strain, a gamma strain, a delta strain, a kappa strain, or an omicron strain) of SARS-CoV-2. For example, by using a JPN/TY/WK-521 strain as a wild strain to be used, and using, as a variant, for example, JPN/QK002/2020 as an alpha strain, JPN/TY8-612-P1/2021 as a beta strain, JPN/TY7-501/2021 as a gamma strain, Japan/TKYTK1734/2021 as a delta strain, Japan/TKYTK5356/2021 as a kappa strain, JPN/TKYX00012/2021 as an omicron BA. 1 strain, and Japan/TKYS02037/2022 as an omicron BA.2 strain, and examining infectivity to a cell line (Matsuyama S et al., PNAS, 117: 7001-7003, 2020) obtained by introducing TMPRSS2 cDNA into Vero E6 cells in the presence of the anti-SARS-CoV-2 antibody, the neutralizing capacity of the antibody can be evaluated (See "7. Evaluation of neutralizing activity" in Examples and Figs. 6 and 7).

Typically, the anti-SARS-CoV-2 antibody or antigen-binding fragment thereof according to the present invention can have 50% infection inhibitory concentration (IC50) of about 200 ng/mL or less, about 150 ng/mL or less, about 100 ng/mL or less, about 90 ng/mL or less, or about 85 ng/mL or less against a wild strain in a system for evaluating SARS-CoV-2 infectivity using the Vero cells.

Alternatively, the anti-SARS-CoV-2 antibody or antigen-binding fragment thereof according to the present invention can have 50% infection inhibitory concentration (IC50) of about 4 μg/mL or less, about 3 μg/mL or less, about 2 μg/mL or less, about 1 μg/mL or less, about 0.5 μg/mL or less, about 0.1 μg/mL or less, or about 0.05 μg/mL or less with respect to an omicron variant, and can have 50% infection inhibitory concentration (IC50) of about 100 ng/mL or less, about 90 ng/mL or less, about 80 ng/mL or less, about 70 ng/mL or less, about 60 ng/mL or less, about 50 ng/mL or less, about 40 ng/mL or less, about 30 ng/mL or less, about 20 ng/mL or less, or about 10 ng/mL or less with respect to a variant other than the omicron strain in the system for evaluating SARS-CoV-2 infectivity using the Vero cells. Non-limiting examples of the variant other than the omicron strain include an alpha strain, a beta strain, a gamma strain, a delta strain, and a kappa strain.

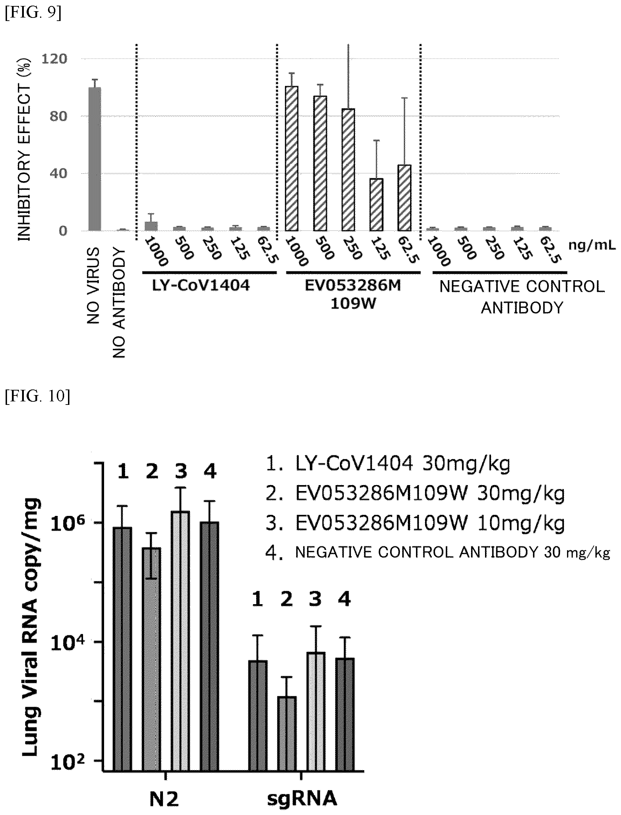

In addition, evaluation of in vivo neutralizing capacity of the anti-SARS-CoV-2 antibody can be performed, for example, using a wild strain and/or a variant of SARS-CoV-2 in a (mammalian) animal model such as a rodent model or a primate model. For example, hCoV-19/Japan/UT-NCD1288-2N/2022 that is an omicron BA.2 strain is used as a variant, and a COVID-19 cynomolgus monkey model reflecting a human disease state (Urano E et al., PNAS, 118 (43): e2104847118, 2021) is used as an animal model. After infection of the animal model with the variant, the anti-SARS-CoV-2 antibody is administered to the animal model, and a disease state thereof and a viral amount thereof are measured, whereby a therapeutic agent effect of the antibody can be evaluated (See "8. Evaluation of drug efficacy using cynomolgus monkey" in Examples and Fig. 10).

Hereinafter, the present invention will be described more specifically with reference to Examples, but the present invention is not limited by these Examples at all.

Examples

Fig. 1 illustrates a flowchart of a procedure of producing the anti-SARS-CoV-2 antibody according to the present invention.

1. Gene cloning of fully human antibody against SARS-CoV-2

Gene cloning of a monoclonal antibody according to the present Example was performed by the following procedure.

1) Peripheral blood of a person who had recovered from SARS-CoV-2 infection was collected.
2) Peripheral blood mononuclear cells (PBMC) were separated by centrifugation.
3) B lymphocytes were separated from PBMC and infected with EBV The infected cells were seeded in a 96-well plate and cultured for about three weeks.
4) Primary screening for the anti-SARS-CoV-2 antibody in a culture supernatant was performed. Screening was performed on an antibody against an S protein trimer (ACROBiosystems), which is a major neutralization target of SARS-CoV-2, and a target antibody-positive well was identified by an ELISA method.
5) Neutralizing activity of each well in which anti-SARS-CoV-2 antibody production was confirmed was measured, and a neutralizing antibody-positive well was identified.
6) Using a culture supernatant of the neutralizing antibody-positive well, an isotype of an antibody produced was confirmed by the ELISA method. That is, an anti-SARS-CoV-2 antibody screening plate in which the S protein trimer was immobilized was used, and an antibody specific to each isotype and subclass was used as a secondary antibody.
7) In some cases, cells of the neutralizing antibody-positive well were seeded on a cell microarray, and by using a slide glass or microbeads in which the S protein trimer was immobilized were used, single cells producing the target antibody were identified.
8) Total-RNA of the antibody-producing cells in the above 5) and 7) was reverse-transcribed using an oligo-dT primer, and an antibody gene was amplified by a PCR method using the obtained cDNA as a template. The primer used for PCR was designed on the basis of a database of cDNA encoding human IgG antibody H and L chains. A 5' terminal side primer and a 3' terminal side primer were designed in order to amplify a region from a translation starting point to a constant region in the H chain and to amplify full-length cDNA of the L chain.

2. Confirmation that obtained antibody gene encodes anti-SARS-CoV-2 antibody

The resulting H chain and L chain genes were each inserted into an expression vector and simultaneously introduced into CHO-K1 cells. The gene transfection was performed with Lipofectamine LTX (Invitrogen) and a plus reagent (Invitrogen) under recommended conditions of the manufacturer. After two days, a culture supernatant was collected, and it was confirmed that an antibody in the culture supernatant bound to an S protein of SARS-CoV-2 by an ELISA method using a multiwell plate in which the S protein trimer was immobilized. For a clone whose binding to the S protein was confirmed, neutralizing activity was measured using the culture supernatant.

3. Determination of amino acid sequence of antibody based on base sequence

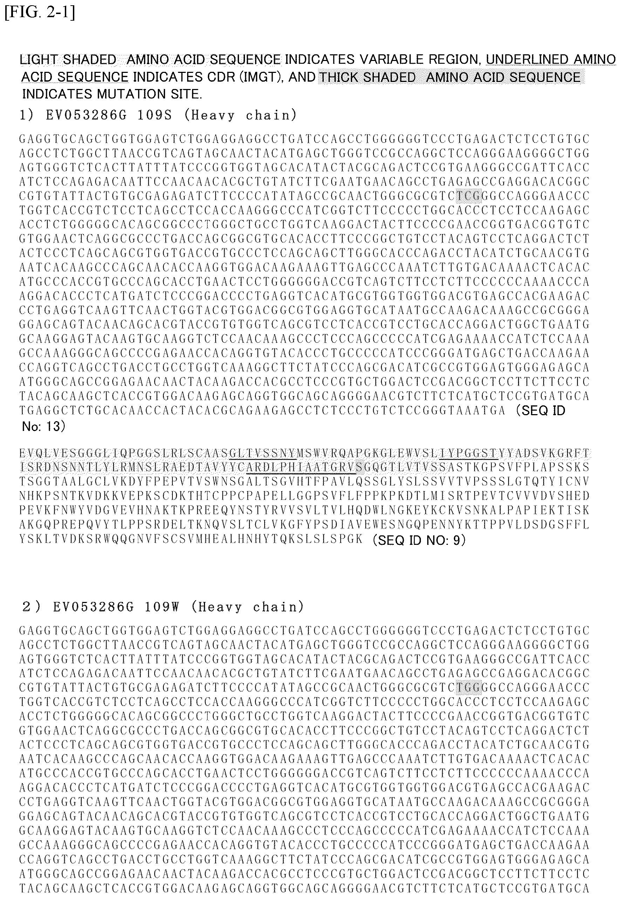

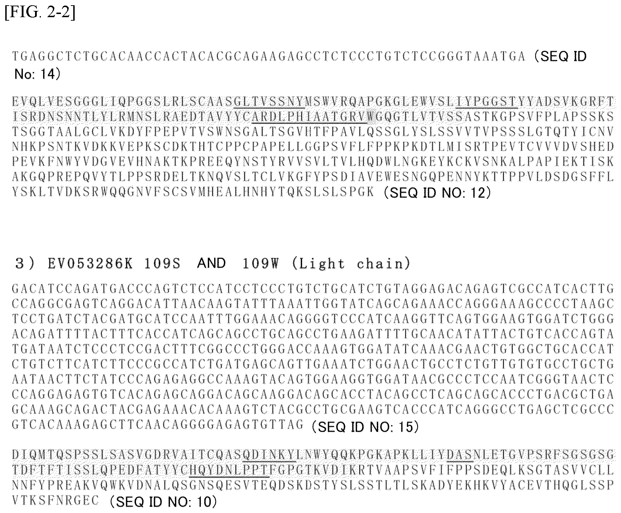

A base sequence of each of the H chain and the L chain was confirmed by an ABI sequencer (ThermoFisher). From the obtained nucleotide sequences, a signal sequence of the antibody, amino acid sequences of the H chain and the L chain, an amino acid sequence of a variable region, and an amino acid sequence of a complementarity determining region (CDR) were determined (Fig. 2). The amino acid sequence of the H chain in the present antibody has a mutation of tryptophan (W) (109W) or serine (S) (109S) at the 109th position of the heavy chain (the first amino acid of FR4). The amino acid sequences of the H chain and L chain, the amino acid sequence of the variable region, and the amino acid sequence of the complementarity determining region (CDR) of the obtained antibody are presented in Table 1. IMGT (http://www.imgt. org/) was used for analysis of CDR. Isotype and subclass were also confirmed.

4. Production and purification of antibody protein

The obtained anti-SARS-CoV-2 neutralizing antibody expression vector was transfected into ExpiCHO cells (Invitrogen), and a culture supernatant was collected. The culture supernatant was subjected to affinity purification with a Protein A column (Cytiva) to obtain a purified antibody. Gene transfection, culture, and purification were performed under recommended conditions of the manufacturer. After purification, a H chain (about 50 kDa) and a L chain (about 25 kDa) of the antibody were confirmed by SDS-PAGE.

5. Evaluation of antigen binding property

Binding properties to the S protein trimer and RBD of a variant were confirmed by an ELISA method using the purified antibody. The anti-SARS-CoV-2 antibody was used at 125 ng/mL. The obtained results are illustrated in Fig. 4. EV053286 109W and EV053286 109S exhibited similar binding properties to the S protein trimer and RBDs of all the measured variants (Fig. 4). In addition, substantially similar results were also obtained for EV053286M 109W in which one amino acid residue (Asp (D): aspartic acid) was added to an N-terminal of the amino acid sequence of the EV053286 109W antibody.

6. Evaluation of antigen affinity of anti-SARS-CoV-2 antibody

Antigen affinity analysis by Biacore T-200 was performed using RBD. Antihuman IgG was immobilized on a sensor chip by a human IgG capture kit (GE Healthcare), and an anti-SARS-CoV-2 antibody (EV053286 109S) was captured to obtain a ligand. As an analyte, RBDs (ACROBiosystems) of a wild strain, an alpha strain, a beta strain, a gamma strain, a delta strain, a kappa strain, an omicron BA.1 strain were used. Results thereof are illustrated in Fig. 5. A $K_D$ value for RBD of the omicron BA.1 strain was $3.22 \times 10^{-8}$ M, and $K_D$ values for RBDs of the other strains were $1.13 \times 10^{-9}$ M or less, indicating very high binding activity.

7. Evaluation of neutralizing activity

Neutralizing activity was confirmed as evaluation of effectiveness of the anti-SARS-CoV-2 antibody. The neutralizing activity was evaluated by a ratio of blocking SARS-CoV-2 virus infection by the antibody. A virus neutralization experiment using the antibody was performed in accordance with the COVID-19 serological test manual: https://www.niid. go.jp/niid/images/pathol/pdf/COVID-19Serology_Ver1.pdf published by the National Institute of Infectious Diseases.

1) Cell culture

A cell line in which TMPRSS2 cDNA was introduced into Vero E6 cells (Matsuyama S et al., PNAS, 117: 7001-7003, 2020) was obtained from the Japanese Collection of Research Bioresources (JCRB) (JCRB1819 VeroE6/TMPRSS2). For maintenance of the cells, a culture solution obtained by adding 400 μg/mL neomycin (G418, Sigma-Aldrich, A1720) to Dulbecco Modified Eagle's medium (DMEM, Nissui 05919) containing fetal calf serum (FCS) at a concentration of 10% was used. The cells were maintained at 37°C under 5% $CO_2$, and for passaging, rinsing was performed twice with a phosphate buffer saline (-) (PBS, Nissui, 05913), and then 1 mL of a 0.25% trypsin/EDTA solution (Lonza, CC-5012) was added thereto per 10 cm plate. The cells were collected with 10% FCS/DMEM and centrifuged at x120 g for five minutes. A supernatant was removed. Thereafter, the cells were suspended in 4% FCS/DMEM, and a cell concentration was counted. Thereafter, the cells were seeded at $10^4$ cells/100 μL per well in a 96-well plate. A viral infection experiment was performed the day after the cell seeding.

2) Viral strain

A wild strain (JPN/TY/WK-521), an alpha strain (JPN/QK002/2020), a beta strain (JPN/TY8-612-P1/2021), a gamma strain (JPN/TY7-501/2021), a delta strain (JPN/TKYTK1734/2021) were obtained from the National Institute of Infectious Diseases, and a kappa strain (Japan/TKYTK5356/2021), an omicron BA.1 strain (Japan/TKYX00012/2021), an omicron BA.2 strain (Japan/TKYS02037/2022), and an omicron BA.5 strain (Japan/TKYS14631/2022) were obtained from Tokyo Metropolitan Institute of Public Health. A virus stock was serially diluted using Opti-MEM (Thermo Fisher Scientific, 31985070) and added to a 96-well plate. One dilution series was used as one set and applied to eight wells. After three to four days, a viral concentration at which half of the eight wells exhibited cytopatheric effects (CPE) was calculated as 50% infectious dose (TCID50, Median tissue culture infectious dose).

3) Production of antibodies Reg10933 and Reg10987 (WO 2021/045836 A1) from Regeneron, and antibody LY-CoV-1404 (US 11447541 B1) from Eli Lilly

For antibodies Reg10933 and Reg10987 from Regeneron, a gene of a full length was synthesized for a light chain and a gene of a variable part region was synthesized for a heavy chain on the basis of a gene sequence described in WO 2021/045836 A1 (See sequence information described in Table ("Table of Exemplary Sequences") in paragraph [000107]) (GeneArt). Each of the synthesized H-chain and L-chain genes was inserted into an expression vector and then transfected into ExpiCHO cells (Invitrogen), and a purified antibody was obtained from a culture supernatant. Production of the expression vector, gene transfection, culture, and purification were performed according to the method described in 1.-4 above. For the antibody LY-CoV-1404 from Eli Lilly, gene synthesis, expression vector production, gene transfection, culture, and purification were performed by GeneScript an amino acid sequence described in US 11447541 B1 (See SEQ ID NOs 5735 and 6736).

4) IC50

A 50% inhibitory concentration (IC50) of each of the purified antibody (EV053286 109S, EV053286 109W, and EV053286M 109W) was evaluated using a 96-well plate (Thermo Fisher Scientific). That is, for an inhibitory effect on each of a wild strain, an alpha strain, a beta strain, a gamma strain, a delta strain, and a kappa strain, an antibody solution diluted with Opti-MEM so as to have a final concentration of 125 ng/mL to 0.24 ng/mL, and of 10 $\mu$g/mL to 19.5 ng/mL for an omicron strain was added to one vertical row of a 96-well plate. Next, a viral liquid prepared so as to have a final MOI of 0.01 was added in an equal amount to each of the 96-well plates, shaken and reacted at 37°C for one hour, and then 100 $\mu$L of the resulting liquid was added to a cell plate. After three days for the wild strain, the alpha strain, the beta strain, the gamma strain, the delta strain, and the kappa strain, and after four days for the omicron strain, 10 $\mu$L of a cell proliferation and toxicity assay kit (FUJIFILM Wako Pure Chemical Corporation) solution was added to each well, and then culture was continued at 37°C. After two to three hours, absorbance at an OD of 450 nm was measured with a plate reader (BIO-RAD, Model 550), a ratio (%) to an uninfected sample was calculated on the basis of a value obtained by subtracting a value of a well to which only the culture solution was added as a background from each value, and neutralizing activity (inhibition ratio) at each antibody concentration was calculated (Figs. 6 and 7). IC50 was calculated from the following calculation formula.

$$IC50 = 10^{\wedge}(LOG(A/B)*(50-C)/(D-C) + LOG(B))$$

A: high concentration sandwiching 50%, B: low concentration sandwiching 50%
C: Inhibition ratio in B, D: Inhibition ratio in A

The antibody of the present invention exhibited an infection inhibitory effect on any of the wild strain, the alpha strain, the beta strain, the gamma strain, the delta strain, and the kappa strain, and the IC50 value thereof was calculated to be 100 ng/mL or less (Fig. 6). Furthermore, the antibody of the present invention also exhibited an infection inhibitory effect on the omicron BA. 1 strain, and the IC50 thereof was calculated to be 3 $\mu$g/mL or less (Fig. 6). The antibody of the present invention exhibited a higher infection inhibitory effect on the omicron BA.2 strain, and the IC50 thereof was calculated to be 0.04 $\mu$g/mL. Efficacy was clear as compared with IC50 = 1.2 $\mu$g/mL of the cocktail antibody from Regeneron (Fig. 7). In addition, substantially similar results to the Figs. 6 and 7 were also obtained for EV053286M 109W in which one amino acid residue (Asp (D): aspartic acid) was added to an N-terminal of the amino acid sequence of the EV053286 109W antibody.

5) Detection of escape variants of cocktail antibody from Regeneron and antibody LY-CoV-1404 from Eli Lilly

An escape variant of a cocktail antibody (Reg10933+Reg10987) from Regeneron was produced using a delta strain. The production was performed twice. Hereinafter, the first production is expressed as EXP-1, and the second production is expressed as EXP-2. For an escape variant of an antibody LY-CoV-1404 from Eli Lilly, production was performed once using an omicron BA.5 strain. A method for the production of escape variants was in accordance with Copin R et al. (2021), Cell 184, 3949-3961. A 10 to 0.016 mg/mL antibody solution and a viral liquid prepared so as to have a final MOI of 1 were mixed and reacted, and then added to host cells. After three days, a culture supernatant was collected.

Among the collected culture supernatants, a culture supernatant having the highest antibody concentration at which infection was completely established, that is, a viral liquid was subjected to a subsequent infection experiment. The viral liquid was prepared so as to have a final dilution magnitude of 20, mixed with a 10 to 0.016 mg/mL antibody solution, and added to new cells. After three days, the viral liquid was collected (second generation). Thereafter, passaging was repeated. For the cocktail antibody from Regeneron, a virus in which infection was established in a 10 mg/mL antibody solution was obtained in the thirteenth generation in EXP-1 and the sixteenth generation in EXP-2. For the escape variant of the antibody LY-CoV-1404 from Eli Lilly, a virus in which infection was established in a 10 mg/mL antibody solution was obtained in the eleventh generation. These were used as escape variants of the antibodies.

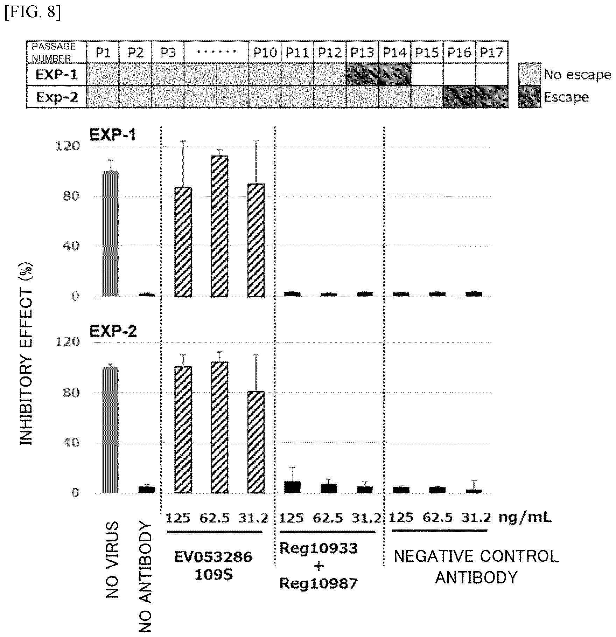

Next, a 125 to 31.25 ng/mL purified antibody (EV053286 109S) solution was mixed with an escape variant of the cocktail antibody from Regeneron to have a final MOI of 0.01, and a 1000 to 62.5 ng/mL purified antibody (EV053286M 109W) solution was mixed with an escape variant of the antibody LY-CoV-1404 from Eli Lilly to have a final MOI of 0.01, and each of the mixtures was added to the host cells. As a result, the antibodies of the present invention suppressed infection of the two Regeneron cocktail antibody escape variants produced in EXP-1 and EXP-2, and infection of the Eli Lilly antibody LY-CoV-1404 escape variant (Figs. 8 and 9).

These characteristics of the monoclonal antibody of the present invention that the monoclonal antibody has a low 50% inhibitory concentration on neutralizing activity of SARS-CoV-2 coronavirus and has neutralizing activity against a plurality of variants including all VOC variants and the Regeneration cocktail antibody escape variants in a small amount are advantageous in use as a medicament (for example, a reduction in load on a patient and a reduction in medical cost).

8. Evaluation of drug efficacy effect by cynomolgus monkey

A COVID-19 cynomolgus monkey model (Urano E et al., PNAS, 118 (43): e2104847118, 2021) was used, and an analysis was performed according to a method of the paper. An omicron BA.2 strain (Japan/UT-NCD1288-2N/2022) was obtained from The Institute of Medical Science, The University of Tokyo. A cynomolgus monkey was inoculated with the omicron BA.2 strain in an amount of $1 \times 10^6$ $TCID_{50}$ (1 ml) through its nose (0.1 ml) and through its respiratory tract (0.9 ml), and on the day after infection, a purified antibody (EV053286M 109W) (10 mg/kg or 30 mg/kg) was intravenously administered to the cynomolgus monkey. A human IgG-$\kappa$ antibody was used as a negative control, and an antibody LY-CoV-1404 from Eli Lilly was used as a positive control (30 mg/kg). Blood and a mucosal swab sample were collected chronologically, and viral shedding and the like were evaluated. On the seventh day of infection, necropsy was performed, and a viral amount in tissues and organs including lungs was evaluated by RT-PCR. When a viral N2 genome amount and an sgRNA amount reflecting viral replication in the lung were analyzed, there was no difference in LY-CoV-1404 from the negative control, whereas the lowest viral amount was observed in a group to which the antibody of the present invention was administered at 30 mg/kg (Fig. 10). Since the antibody of the present invention exhibits suppression of SARS-CoV-2 coronavirus proliferation in a primate model such as a cynomolgus monkey model, an effect of preventing or treating a disease in which SARS-CoV-2 is involved is expected also in humans.

Industrial Applicability

The anti-SARS-CoV-2 antibody of the present invention is useful for application in a field of a pharmaceutical composition and the like for preventing or treating a disease in which SARS-CoV-2 is involved.

[Brief Description of Sequences]

> HCVR of EV053286 109S

EVQLVESGGGLIQPGGSLRLSCAASGLTVSSNYMSWVRQAPGKGLEWVSLIYPG

GSTYYADSVKGRFTISRDNSNNTLYLRMNSLRAEDTAVYYCARDLPHIAATGRV

SGQGTLVTVSS (SEQ ID NO: 1)

> EV053286 109S, EV053286 109W, and EV053286M 109W
HCDR1GLTVSSNY (SEQ ID NO: 2)

> EV053286 109S, EV053286 109W, and EV053286M 109W
HCDR2IYPGGST (SEQ ID NO: 3)

> EV053286 109S, EV053286 109W, and EV053286M 109W
HCDR3ARDLPHIAATGRV (SEQ ID NO: 4)

> LCVR of EV053286 109S and EV053286 109W

DIQMTQSPSSLSASVGDRVAITCQAS<u>QDINKY</u>LNWYQQKPGKAPKLLIY<u>DAS</u>NL

ETGVPSRFSGSGSGTDFTFTISSLQPEDFATYYC<u>HQYDNLPPT</u>FGPGTKVDIK

(SEQ ID NO: 5)

> LCDR1 of EV053286 109S and EV053286 109W
QDINKY (SEQ ID NO: 6)

> LCDR2 of EV053286 109S and EV053286 109W
DAS (SEQ ID NO: 7)

> LCDR3 of EV053286 109S and EV053286 109W
HQYDNLPPT (SEQ ID NO: 8)

> HC ofEV053286 109S

EVQLVESGGGLIQPGGSLRLSCAAS<u>GLTVSSNY</u>MSWVRQAPGKGLEWVSL<u>IYPG</u>

<u>GST</u>YYADSVKGRFTISRDNSNNTLYLRMNSLRAEDTAVYYC<u>ARDLPHIAATGRV</u>

SGQGTLVTVSSASTKGPSVFPLAPSSKSTSGGTAALGCLVKDYFPEPVTVSWNSG

ALTSGVHTFPAVLQSSGLYSLSSVVTVPSSSLGTQTYICNVNHKPSNTKVDKKVE

PKSCDKTHTCPPCPAPELLGGPSVFLFPPKPKDTLMISRTPEVTCVVVDVSHEDP

EVKFNWYVDGVEVHNAKTKPREEQYNSTYRVVSVLTVLHQDWLNGKEYKCK

VSNKALPAPIEKTISKAKGQPREPQVYTLPPSRDELTKNQVSLTCLVKGFYPSDIA

VEWESNGQPENNYKTTPPVLDSDGSFFLYSKLTVDKSRWQQGNVFSCSVMHEA

LHNHYTQKSLSLSPGK (SEQ ID NO: 9)

> LC of EV053286 109S and EV053286 109W

DIQMTQSPSSLSASVGDRVAITCQAS<u>QDINKY</u>LNWYQQKPGKAPKLLIY<u>DASNL</u>
ETGVPSRFSGSGSGTDFTFTISSLQPEDFATYYC<u>HQYDNLPPT</u>FGPGTKVDIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC (SEQ
ID NO: 10)

> HCVR of EV053286 109W

EVQLVESGGGLIQPGGSLRLSCAAS<u>GLTVSSNY</u>MSWVRQAPGKGLEWVSL<u>IYPG
GST</u>YYADSVKGRFTISRDNSNNTLYLRMNSLRAEDTAVYYC<u>ARDLPHIAATGRV</u>
WGQGTLVTVSS (SEQ ID NO: 11)

> HC ofEV053286 109W

EVQLVESGGGLIQPGGSLRLSCAAS<u>GLTVSSNY</u>MSWVRQAPGKGLEWVSL<u>IYPG
GST</u>YYADSVKGRFTISRDNSNNTLYLRMNSLRAEDTAVYYC<u>ARDLPHIAATGRV</u>
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK (SEQ ID NO: 12)

> DNA encoding HC of EV053286 109S (not containing signal sequence)

GAGGTGCAGCTGGTGGAGTCTGGAGGAGGCCTGATCCAGCCTGGGGGGTCC

CTGAGACTCTCCTGTGCAGCCTCTGGCTTAACCGTCAGTAGCAACTACATGA

GCTGGGTCCGCCAGGCTCCAGGGAAGGGGCTGGAGTGGGTCTCACTTATTTA

TCCCGGTGGTAGCACATACTACGCAGACTCCGTGAAGGGCCGATTCACCATC

TCCAGAGACAATTCCAACAACACGCTGTATCTTCGAATGAACAGCCTGAGAG

CCGAGGACACGGCCGTGTATTACTGTGCGAGAGATCTTCCCCATATAGCCGCA

ACTGGGCGCGTCTCGGGCCAGGGAACCCTGGTCACCGTCTCCTCAGCCTCCA

CCAAGGGCCCATCGGTCTTCCCCCTGGCACCCTCCTCCAAGAGCACCTCTGG

GGGCACAGCGGCCCTGGGCTGCCTGGTCAAGGACTACTTCCCCGAACCGGT

GACGGTGTCGTGGAACTCAGGCGCCCTGACCAGCGGCGTGCACACCTTCCC

GGCTGTCCTACAGTCCTCAGGACTCTACTCCCTCAGCAGCGTGGTGACCGTG

CCCTCCAGCAGCTTGGGCACCCAGACCTACATCTGCAACGTGAATCACAAGC

CCAGCAACACCAAGGTGGACAAGAAAGTTGAGCCCAAATCTTGTGACAAAA

CTCACACATGCCCACCGTGCCCAGCACCTGAACTCCTGGGGGGACCGTCAGT

CTTCCTCTTCCCCCCAAAACCCAAGGACACCCTCATGATCTCCGGACCCCT

GAGGTCACATGCGTGGTGGTGGACGTGAGCCACGAAGACCCTGAGGTCAAG

TTCAACTGGTACGTGGACGGCGTGGAGGTGCATAATGCCAAGACAAAGCCG

CGGGAGGAGCAGTACAACAGCACGTACCGTGTGGTCAGCGTCCTCACCGTC

CTGCACCAGGACTGGCTGAATGGCAAGGAGTACAAGTGCAAGGTCTCCAAC

AAAGCCCTCCCAGCCCCCATCGAGAAAACCATCTCCAAAGCCAAAGGGCAG

CCCCGAGAACCACAGGTGTACACCCTGCCCCCATCCCGGGATGAGCTGACCA

AGAACCAGGTCAGCCTGACCTGCCTGGTCAAAGGCTTCTATCCCAGCGACAT

CGCCGTGGAGTGGGAGAGCAATGGGCAGCCGGAGAACAACTACAAGACCA

CGCCTCCCGTGCTGGACTCCGACGGCTCCTTCTTCCTCTACAGCAAGCTCAC

CGTGGACAAGAGCAGGTGGCAGCAGGGGAACGTCTTCTCATGCTCCGTGAT

GCATGAGGCTCTGCACAACCACTACACGCAGAAGAGCCTCTCCCTGTCTCCG

GGTAAATGA (SEQ ID NO: 13)

> DNA encoding HC of EV053286 109W (not containing signal sequence)

GAGGTGCAGCTGGTGGAGTCTGGAGGAGGCCTGATCCAGCCTGGGGGGGTCC

CTGAGACTCTCCTGTGCAGCCTCTGGCTTAACCGTCAGTAGCAACTACATGA

GCTGGGTCCGCCAGGCTCCAGGGAAGGGGCTGGAGTGGGTCTCACTTATTTA

TCCCGGTGGTAGCACATACTACGCAGACTCCGTGAAGGGCCGATTCACCATC

TCCAGAGACAATTCCAACAACACGCTGTATCTTCGAATGAACAGCCTGAGAG

CCGAGGACACGGCCGTGTATTACTGTGCGAGAGATCTTCCCCATATAGCCGCA

ACTGGGCGCGTCTGGGGCCAGGGAACCCTGGTCACCGTCTCCTCAGCCTCC

ACCAAGGGCCCATCGGTCTTCCCCCTGGCACCCTCCTCCAAGAGCACCTCTG

GGGGCACAGCGGCCCTGGGCTGCCTGGTCAAGGACTACTTCCCCGAACCGG

TGACGGTGTCGTGGAACTCAGGCGCCCTGACCAGCGGCGTGCACACCTTCC

CGGCTGTCCTACAGTCCTCAGGACTCTACTCCCTCAGCAGCGTGGTGACCGT

GCCCTCCAGCAGCTTGGGCACCCAGACCTACATCTGCAACGTGAATCACAAG

CCCAGCAACACCAAGGTGGACAAGAAAGTTGAGCCCAAATCTTGTGACAAA

ACTCACACATGCCCACCGTGCCCAGCACCTGAACTCCTGGGGGGACCGTCA

GTCTTCCTCTTCCCCCCAAAACCCAAGGACACCCTCATGATCTCCCGGACCC

CTGAGGTCACATGCGTGGTGGTGGACGTGAGCCACGAAGACCCTGAGGTCA

AGTTCAACTGGTACGTGGACGGCGTGGAGGTGCATAATGCCAAGACAAAGC

CGCGGGAGGAGCAGTACAACAGCACGTACCGTGTGGTCAGCGTCCTCACCG

TCCTGCACCAGGACTGGCTGAATGGCAAGGAGTACAAGTGCAAGGTCTCCA

ACAAAGCCCTCCCAGCCCCCATCGAGAAAACCATCTCCAAAGCCAAAGGGC

AGCCCCGAGAACCACAGGTGTACACCCTGCCCCCATCCCGGGATGAGCTGAC

CAAGAACCAGGTCAGCCTGACCTGCCTGGTCAAAGGCTTCTATCCCAGCGAC

ATCGCCGTGGAGTGGGAGAGCAATGGGCAGCCGGAGAACAACTACAAGACC

ACGCCTCCCGTGCTGGACTCCGACGGCTCCTTCTTCCTCTACAGCAAGCTCA

CCGTGGACAAGAGCAGGTGGCAGCAGGGGAACGTCTTCTCATGCTCCGTGA

TGCATGAGGCTCTGCACAACCACTACACGCAGAAGAGCCTCTCCCTGTCTCC

GGGTAAATGA (SEQ ID NO: 14)

> DNA encoding LC of EV053286 (not containing signal sequence)

GACATCCAGATGACCCAGTCTCCATCCTCCCTGTCTGCATCTGTAGGAGACAG

AGTCGCCATCACTTGCCAGGCGAGTCAGGACATTAACAAGTATTTAAATTGGT

ATCAGCAGAAACCAGGGAAAGCCCCTAAGCTCCTGATCTACGATGCATCCAA

TTTGGAAACAGGGGTCCCATCAAGGTTCAGTGGAAGTGGATCTGGGACAGA

TTTTACTTTCACCATCAGCAGCCTGCAGCCTGAAGATTTTGCAACATATTACT

GTCACCAGTATGATAATCTCCCTCCGACTTTCGGCCCTGGGACCAAAGTGGAT

ATCAAACGAACTGTGGCTGCACCATCTGTCTTCATCTTCCCGCCATCTGATGA

GCAGTTGAAATCTGGAACTGCCTCTGTTGTGTGCCTGCTGAATAACTTCTATC

CCAGAGAGGCCAAAGTACAGTGGAAGGTGGATAACGCCCTCCAATCGGGTA

ACTCCCAGGAGAGTGTCACAGAGCAGGACAGCAAGGACAGCACCTACAGCC

TCAGCAGCACCCTGACGCTGAGCAAAGCAGACTACGAGAACACAAAGTCT

ACGCCTGCGAAGTCACCCATCAGGGCCTGAGCTCGCCCGTCACAAAGAGCT

TCAACAGGGGAGAGTGTTAG (SEQ ID NO: 15)

> DNA encoding HC of EV053286M 109W (not containing signal sequence)

GACGAGGTGCAGCTGGTGGAGTCTGGAGGAGGCCTGATCCAGCCTGGGGGG

TCCCTGAGACTCTCCTGTGCAGCCTCTGGCTTAACCGTCAGTAGCAACTACAT

GAGCTGGGTCCGCCAGGCTCCAGGGAAGGGGCTGGAGTGGGTCTCACTTAT

TTATCCCGGTGGTAGCACATACTACGCAGACTCCGTGAAGGGCCGATTCACC

ATCTCCAGAGACAATTCCAACAACACGCTGTATCTTCGAATGAACAGCCTGA

GAGCCGAGGACACGGCCGTGTATTACTGTGCGAGAGATCTTCCCCATATAGC

CGCAACTGGGCGCGTCTGGGGCCAGGGAACCCTGGTCACCGTCTCCTCAGC

CTCCACCAAGGGCCCATCGGTCTTCCCCCTGGCACCCTCCTCCAAGAGCACC

TCTGGGGGCACAGCGGCCCTGGGCTGCCTGGTCAAGGACTACTTCCCCGAA

CCGGTGACGGTGTCGTGGAACTCAGGCGCCCTGACCAGCGGCGTGCACACC

TTCCCGGCTGTCCTACAGTCCTCAGGACTCTACTCCCTCAGCAGCGTGGTGA

CCGTGCCCTCCAGCAGCTTGGGCACCCAGACCTACATCTGCAACGTGAATCA

CAAGCCCAGCAACACCAAGGTGGACAAGAAAGTTGAGCCCAAATCTTGTGA

CAAAACTCACACATGCCCACCGTGCCCAGCACCTGAACTCCTGGGGGGACC

GTCAGTCTTCCTCTTCCCCCCAAAACCCAAGGACACCCTCATGATCTCCCGG

ACCCCTGAGGTCACATGCGTGGTGGTGGACGTGAGCCACGAAGACCCTGAG

GTCAAGTTCAACTGGTACGTGGACGGCGTGGAGGTGCATAATGCCAAGACA

AAGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAACCATCTCCAAAGCCAAA
GGGCAGCCCCGAGAACCACAGGTGTACACCCTGCCCCCATCCCGGGATGAG
CTGACCAAGAACCAGGTCAGCCTGACCTGCCTGGTCAAAGGCTTCTATCCCA
GCGACATCGCCGTGGAGTGGGAGAGCAATGGGCAGCCGGAGAACAACTACA
AGACCACGCCTCCCGTGCTGGACTCCGACGGCTCCTTCTTCCTCTACAGCAA
GCTCACCGTGGACAAGAGCAGGTGGCAGCAGGGGAACGTCTTCTCATGCTC
CGTGATGCATGAGGCTCTGCACAACCACTACACGCAGAAGAGCCTCTCCCTG
TCTCCGGGTAAATGA (SEQ ID NO: 16)

> HC ofEV053286M 109W

DEVQLVESGGGLIQPGGSLRLSCAAS<u>GLTVSSNY</u>MSWVRQAPGKGLEWVSL<u>IYP</u>
<u>GGST</u>YYADSVKGRFTISRDNSNNTLYLRMNSLRAEDTAVYYC<u>ARDLPHIAATGR</u>
<u>V</u>WGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK (SEQ ID NO: 17)

> DNA encoding LC of EV053286M 109W (not containing signal sequence)

GACGACATCCAGATGACCCAGTCTCCATCCTCCCTGTCTGCATCTGTAGGAGA
CAGAGTCGCCATCACTTGCCAGGCGAGTCAGGACATTAACAAGTATTTAAAT

TGGTATCAGCAGAAACCAGGGAAAGCCCCTAAGCTCCTGATCTACGATGCAT CCAATTTGGAAACAGGGGTCCCATCAAGGTTCAGTGGAAGTGGATCTGGGA CAGATTTTACTTTCACCATCAGCAGCCTGCAGCCTGAAGATTTTGCAACATAT TACTGTCACCAGTATGATAATCCCTCCGACTTTCGGCCCTGGGACCAAAGT GGATATCAAACGAACTGTGGCTGCACCATCTGTCTTCATCTTCCCGCCATCTG ATGAGCAGTTGAAATCTGGAACTGCCTCTGTTGTGTGCCTGCTGAATAACTTC TATCCCAGAGAGGCCAAAGTACAGTGGAAGGTGGATAACGCCCTCCAATCGG GTAACTCCCAGGAGAGTGTCACAGAGCAGGACAGCAAGGACAGCACCTACA GCCTCAGCAGCACCCTGACGCTGAGCAAAGCAGACTACGAGAACACAAAG TCTACGCCTGCGAAGTCACCCATCAGGGCCTGAGCTCGCCCGTCACAAAGAG CTTCAACAGGGGAGAGTGTTAG (SEQ ID NO: 18)

> LC of EV053286M 109W

DDIQMTQSPSSLSASVGDRVAITCQAS<u>QDINKY</u>LNWYQQKPGKAPKLLIY<u>DAS</u>N LETGVPSRFSGSGSGTDFTFTISSLQPEDFATYYC<u>HQYDNLPPT</u>FGPGTKVDIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC
(SEQ ID NO: 19)

> HCVR of EV053286M 109W

DEVQLVESGGGLIQPGGSLRLSCAAS<u>GLTVSSNY</u>MSWVRQAPGKGLEWVSL<u>IYP GGST</u>YYADSVKGRFTISRDNSNNTLYLRMNSLRAEDTAVYYC<u>ARDLPHIAATGR V</u>WGQGTLVTVSS (SEQ ID NO: 20)

> LCVR of EV053286M 109W

DDIQMTQSPSSLSASVGDRVAITCQAS<u>QDINKY</u>LNWYQQKPGKAPKLLIY<u>DAS</u>N LETGVPSRFSGSGSGTDFTFTISSLQPEDFATYYC<u>HQYDNLPPT</u>FGPGTKVDIK
(SEQ ID NO: 21)

Claims

1. A monoclonal antibody against SARS-CoV-2 coronavirus and an antigen-binding fragment thereof, wherein the monoclonal antibody binds to a spike protein of SARS-CoV-2 coronavirus to neutralize viral activity.

2. The antibody or the antigen-binding fragment thereof according to claim 1, comprising:

    a heavy chain variable region including an amino acid sequence of SEQ ID NO: 1, SEQ ID NO: 11, or SEQ ID NO: 20, or an amino acid sequence having 90% or more of identity to the amino acid sequence of SEQ ID NO: 1, SEQ ID NO: 11, or SEQ ID NO: 20; and
    a light chain variable region including an amino acid sequence of SEQ ID NO: 5 or SEQ ID NO: 21, or an amino acid sequence having 90% or more of identity to the amino acid sequence of SEQ ID NO: 5 or SEQ ID NO: 21.

3. The antibody or the antigen-binding fragment thereof according to claim 1 or 2, comprising:

    a heavy chain including an amino acid sequence of SEQ ID NO: 9, SEQ ID NO: 12, or SEQ ID NO: 17, or an amino acid sequence having 90% or more of identity to the amino acid sequence of SEQ ID NO: 9, SEQ ID NO: 12, or SEQ ID NO: 17; and
    a light chain including an amino acid sequence of SEQ ID NO: 10 or SEQ ID NO: 19, or an amino acid sequence having 90% or more of identity to the amino acid sequence of SEQ ID NO: 10 or SEQ ID NO: 19.

4. The antibody or the antigen-binding fragment thereof according to any one of claims 1 to 3, wherein

    (i) a heavy chain variable region includes:

        (a) an amino acid sequence of a heavy chain CDR1 including an amino acid sequence of SEQ ID NO: 2,
        (b) an amino acid sequence of a heavy chain CDR2 including an amino acid sequence of SEQ ID NO: 3, and
        (c) an amino acid sequence of a heavy chain CDR3 including an amino acid sequence of SEQ ID NO: 4, and

    (ii) a light chain variable region includes:

        (a) an amino acid sequence of a light chain CDR1 including an amino acid sequence of SEQ ID NO: 6,
        (b) an amino acid sequence of a light chain CDR2 including an amino acid sequence of SEQ ID NO: 7, and
        (c) an amino acid sequence of a light chain CDR3 including an amino acid sequence of SEQ ID NO: 8.

5. The antibody or the antigen-binding fragment thereof according to any one of claims 1 to 4, wherein neutralizing activity (IC50) against either or both of a wild strain and a variant including an alpha strain, a beta strain, a gamma strain, a delta type, and a kappa strain of SARS-CoV-2 is about 100 ng/mL or less, and/or neutralizing activity against an omicron strain of SARS-CoV-2 is about 3 $\mu$g/mL or less.

6. The antibody or the antigen-binding fragment thereof according to any one of claims 1 to 5, having activity for neutralizing escape variants of a cocktail antibody containing antibodies specified by each of mAb10933 and mAb10987 in WO 2021/045836 or an equivalent thereof and an antibody exhibiting activity on strains from a wild strain to omicron BA.5, specified to be 1404 in US 11447541 B1.

7. The antibody or the antigen-binding fragment thereof according to any one of claims 1 to 6, wherein a $K_D$ value for RBD of each of a wild strain, an alpha strain, a beta strain, a gamma strain, a delta type, and a kappa strain is about 1.2 x $10^{-9}$ M or less, and/or a $K_D$ value for RBD of an omicron strain is about 3.3 x $10^{-8}$ M.

8. The antibody or the antigen-binding fragment thereof according to any one of claims 1 to 7, comprising

    (i) an antibody or an antigen-binding fragment thereof that suppresses SARS-CoV-2 coronavirus proliferation in a primate model, or
    (ii) a heavy chain consisting of an amino acid sequence of SEQ ID NO: 9, SEQ ID NO: 12, or SEQ ID NO: 17 and a light chain consisting of an amino acid sequence of SEQ ID NO: 10 or SEQ ID NO: 19.

9. A pharmaceutical composition for treating or preventing a SARS-CoV-2 coronavirus infectious disease, comprising the antibody or the antigen-binding fragment thereof according to any one of claims 1 to 8 and a pharmaceutically acceptable carrier.

**10.**

(1) Use of the antibody or the antigen-binding fragment thereof according to any one of claims 1 to 9 in treatment or prevention of a SARS-CoV-2 coronavirus infectious disease,
(2) use of the antibody or the antigen-binding fragment thereof according to any one of claims 1 to 7 in production of a medicament for treating or preventing a SARS-CoV-2 coronavirus infectious disease, or
(3) a method for treating or preventing a SARS-CoV-2 coronavirus infectious disease, comprising administering the antibody or the antigen-binding fragment thereof according to any one of claims 1 to 8 to a subject or a patient in need thereof.

**11.** The pharmaceutical composition according to claim 9 or the use or method according to claim 9, wherein the SARS-CoV-2 is a variant.

**12.** The pharmaceutical composition, use, or method according to claim 11, wherein the variant is an alpha strain, a beta strain, a gamma strain, a delta strain, a kappa strain, or an omicron strain.

**13.** An isolated nucleic acid molecule encoding an amino acid sequence of the antibody or the antigen-binding fragment thereof according to any one of claims 1 to 8, or an isolated nucleic acid molecule that hybridizes with any of these nucleic acid molecules under highly stringent conditions.

**14.** A recombinant expression vector into which the isolated nucleic acid molecule according to claim 13 is incorporated.

**15.** An isolated host cell into which the recombinant expression vector according to claim 14 is introduced.

**16.** A method for producing the antibody or the antigen-binding fragment thereof according to any one of claims 1 to 8, comprising culturing the host cell according to claim 15 and purifying an antibody from the cultured host cell.

[FIG. 1]

```
┌─────────────────────────────────────────────┐
│ PERIPHERAL BLOOD OF PERSON WHO HAS           │
│ RECOVERED FROM SARS-CoV-2 INFECTION          │
└─────────────────────────────────────────────┘
                    ⇩
┌─────────────────────────────────────────────┐
│              SEPARATE PBMC                    │
│          SEPARATE B LYMPHOCYTE                │
│             INFECTED WITH EBV                 │
└─────────────────────────────────────────────┘
┌─────────────────────────────────────────────┐
│        SEEDED IN 96-WELL PLATE AND           │
│         CULTURED (~ THREE WEEKS)             │
└─────────────────────────────────────────────┘
              │    ┌─────────────────────────────────────────┐
              │    │ DETECT S PROTEIN TRIMER-BINDING          │
              │    │ ANTIBODY-POSITIVE WELL                   │
              │    ├─────────────────────────────────────────┤
              │    │ IDENTIFY NEUTRALIZING                    │
              │    │ ANTIBODY-POSITIVE WELL                   │
              ⇩    └─────────────────────────────────────────┘
┌─────────────────────────────────────────────┐
│   IDENTIFY ANTIGEN BINDING-POSITIVE CELL     │
└─────────────────────────────────────────────┘
                    ⇩
┌─────────────────────────────────────────────┐
│              GENE CLONING                     │
└─────────────────────────────────────────────┘
                    ⇩
┌─────────────────────────────────────────────┐
│              TRANSFECTION                     │
│       CONFIRM NEUTRALIZING ACTIVITY          │
└─────────────────────────────────────────────┘
┌─────────────────────────────────────────────┐
│              SEQUENCE                         │
└─────────────────────────────────────────────┘
┌─────────────────────────────────────────────┐
│        TRANSFECTION (LARGE SCALE)            │
│            PURIFY ANTIBODY                    │
└─────────────────────────────────────────────┘
```

[FIG. 2-1]

LIGHT SHADED AMINO ACID SEQUENCE INDICATES VARIABLE REGION, UNDERLINED AMINO ACID SEQUENCE INDICATES CDR (IMGT), AND THICK SHADED AMINO ACID SEQUENCE INDICATES MUTATION SITE.

1) EV053286G 109S (Heavy chain)

```
GAGGTGCAGCTGGTGGAGTCTGGAGGAGGCCTGATCCAGCCTGGGGGGTCCCTGAGACTCTCCTGTGC
AGCCTCTGGCTTAACCGTCAGTAGCAACTACATGAGCTGGGTCCGCCAGGCTCCAGGGAAGGGGCTGG
AGTGGGTCTCACTTATTTATCCCGGTGGTAGCACATACTACGCAGACTCCGTGAAGGGCCGATTCACC
ATCTCCAGAGACAATTCCAACAACACGCTGTATCTTCGAATGAACAGCCTGAGAGCCGAGGACACGGC
CGTGTATTACTGTGCGAGAGATCTTCCCCATATAGCCGCAACTGGGCGCGTCTCGGGCCAGGGAACCC
TGGTCACCGTCTCCTCAGCCTCCACCAAGGGCCCATCGGTCTTCCCCCTGGCACCCTCCTCCAAGAGC
ACCTCTGGGGGCACAGCGGCCCTGGGCTGCCTGGTCAAGGACTACTTCCCCGAACCGGTGACGGTGTC
GTGGAACTCAGGCGCCCTGACCAGCGGCGTGCACACCTTCCCGGCTGTCCTACAGTCCTCAGGACTCT
ACTCCCTCAGCAGCGTGGTGACCGTGCCCTCCAGCAGCTTGGGCACCCAGACCTACATCTGCAACGTG
AATCACAAGCCCAGCAACACCAAGGTGGACAAGAAAGTTGAGCCCAAATCTTGTGACAAAACTCACAC
ATGCCCACCGTGCCCAGCACCTGAACTCCTGGGGGGACCGTCAGTCTTCCTCTTCCCCCCAAAACCCA
AGGACACCCTCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAAGCCGCGGGA
GGAGCAGTACAACAGCACGTACCGTGTGGTCAGCGTCCTCACCGTCCTGCACCAGGACTGGCTGAATG
GCAAGGAGTACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATCCCGGGATGAGCTGACCAAGAA
CCAGGTCAGCCTGACCTGCCTGGTCAAAGGCTTCTATCCCAGCGACATCGCCGTGGAGTGGGAGAGCA
ATGGGCAGCCGGAGAACAACTACAAGACCACGCCTCCCGTGCTGGACTCCGACGGCTCCTTCTTCCTC
TACAGCAAGCTCACCGTGGACAAGAGCAGGTGGCAGCAGGGGAACGTCTTCTCATGCTCCGTGATGCA
TGAGGCTCTGCACAACCACTACACGCAGAAGAGCCTCTCCCTGTCTCCGGGTAAATGA (SEQ ID
No: 13)
```

```
EVQLVESGGGLIQPGGSLRLSCAASGLTVSSNYMSWVRQAPGKGLEWVSLIYPGGSTYYADSVKGRFT
ISRDNSNNTLYLRMNSLRAEDTAVYYCARDLPHIAATGRVSGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO: 9)
```

2) EV053286G 109W (Heavy chain)

```
GAGGTGCAGCTGGTGGAGTCTGGAGGAGGCCTGATCCAGCCTGGGGGGTCCCTGAGACTCTCCTGTGC
AGCCTCTGGCTTAACCGTCAGTAGCAACTACATGAGCTGGGTCCGCCAGGCTCCAGGGAAGGGGCTGG
AGTGGGTCTCACTTATTTATCCCGGTGGTAGCACATACTACGCAGACTCCGTGAAGGGCCGATTCACC
ATCTCCAGAGACAATTCCAACAACACGCTGTATCTTCGAATGAACAGCCTGAGAGCCGAGGACACGGC
CGTGTATTACTGTGCGAGAGATCTTCCCCATATAGCCGCAACTGGGCGCGTCTGGGGCCAGGGAACCC
TGGTCACCGTCTCCTCAGCCTCCACCAAGGGCCCATCGGTCTTCCCCCTGGCACCCTCCTCCAAGAGC
ACCTCTGGGGGCACAGCGGCCCTGGGCTGCCTGGTCAAGGACTACTTCCCCGAACCGGTGACGGTGTC
GTGGAACTCAGGCGCCCTGACCAGCGGCGTGCACACCTTCCCGGCTGTCCTACAGTCCTCAGGACTCT
ACTCCCTCAGCAGCGTGGTGACCGTGCCCTCCAGCAGCTTGGGCACCCAGACCTACATCTGCAACGTG
AATCACAAGCCCAGCAACACCAAGGTGGACAAGAAAGTTGAGCCCAAATCTTGTGACAAAACTCACAC
ATGCCCACCGTGCCCAGCACCTGAACTCCTGGGGGGACCGTCAGTCTTCCTCTTCCCCCCAAAACCCA
AGGACACCCTCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAAGCCGCGGGA
GGAGCAGTACAACAGCACGTACCGTGTGGTCAGCGTCCTCACCGTCCTGCACCAGGACTGGCTGAATG
GCAAGGAGTACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATCCCGGGATGAGCTGACCAAGAA
CCAGGTCAGCCTGACCTGCCTGGTCAAAGGCTTCTATCCCAGCGACATCGCCGTGGAGTGGGAGAGCA
ATGGGCAGCCGGAGAACAACTACAAGACCACGCCTCCCGTGCTGGACTCCGACGGCTCCTTCTTCCTC
TACAGCAAGCTCACCGTGGACAAGAGCAGGTGGCAGCAGGGGAACGTCTTCTCATGCTCCGTGATGCA
```

[FIG. 2-2]

TGAGGCTCTGCACAACCACTACACGCAGAAGAGCCTCTCCCTGTCTCCGGGTAAATGA (SEQ ID No: 14)

EVQLVESGGGLIQPGGSLRLSCAASGLTVSSNYMSWVRQAPGKGLEWVSLIYPGGSTYYADSVKGRFT
ISRDNSNNTLYLRMNSLRAEDTAVYYCARDLPHIAATGRVWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO: 12)

3) EV053286K 109S AND 109W (Light chain)

GACATCCAGATGACCCAGTCTCCATCCTCCCTGTCTGCATCTGTAGGAGACAGAGTCGCCATCACTTG
CCAGGCGAGTCAGGACATTAACAAGTATTTAAATTGGTATCAGCAGAAACCAGGGAAAGCCCCTAAGC
TCCTGATCTACGATGCATCCAATTTGGAAACAGGGGTCCCATCAAGGTTCAGTGGAAGTGGATCTGGG
ACAGATTTTACTTTCACCATCAGCAGCCTGCAGCCTGAAGATTTTGCAACATATTACTGTCACCAGTA
TGATAATCTCCCTCCGACTTTCGGCCCTGGGACCAAAGTGGATATCAAACGAACTGTGGCTGCACCAT
CTGTCTTCATCTTCCCGCCATCTGATGAGCAGTTGAAATCTGGAACTGCCTCTGTTGTGTGCCTGCTG
AATAACTTCTATCCCAGAGAGGCCAAAGTACAGTGGAAGGTGGATAACGCCCTCCAATCGGGTAACTC
CCAGGAGAGTGTCACAGAGCAGGACAGCAAGGACAGCACCTACAGCCTCAGCAGCACCCTGACGCTGA
GCAAAGCAGACTACGAGAAACACAAAGTCTACGCCTGCGAAGTCACCCATCAGGGCCTGAGCTCGCCC
GTCACAAAGAGCTTCAACAGGGGAGAGTGTTAG (SEQ ID NO: 15)

DIQMTQSPSSLSASVGDRVAITCQASQDINKYLNWYQQKPGKAPKLLIYDASNLETGVPSRFSGSGSG
TDFTFTISSLQPEDFATYYCHQYDNLPPTFGPGTKVDIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC (SEQ ID NO: 10)

[FIG. 3]

UNDERLINED AMINO ACID SEQUENCE INDICATES CDR (IMGT).


1) EV053286M 109W (Heavy chain)

GACGAGGTGCAGCTGGTGGAGTCTGGAGGAGGCCTGATCCAGCCTGGGGGGTCCCTGAGACTCTCCTGTGCAGCCTC
TGGCTTAACCGTCAGTAGCAACTACATGAGCTGGGTCCGCCAGGCTCCAGGGAAGGGGCTGGAGTGGGTCTCACTTA
TTTATCCCGGTGGTAGCACATACTACGCAGACTCCGTGAAGGGCCGATTCACCATCTCCAGAGACAATTCCAACAAC
ACGCTGTATCTTCGAATGAACAGCCTGAGAGCCGAGGACACGGCCGTGTATTACTGTGCGAGAGATCTTCCCCATAT
AGCCGCAACTGGGCGCGTCTGGGGCCAGGGAACCCTGGTCACCGTCTCCTCAGCCTCCACCAAGGGCCCATCGGTCT
TCCCCCTGGCACCCTCCTCCAAGAGCACCTCTGGGGGCACAGCGGCCCTGGGCTGCCTGGTCAAGGACTACTTCCCC
GAACCGGTGACGGTGTCGTGGAACTCAGGCGCCCTGACCAGCGGCGTGCACACCTTCCCGGCTGTCCTACAGTCCTC
AGGACTCTACTCCCTCAGCAGCGTGGTGACCGTGCCCTCCAGCAGCTTGGGCACCCAGACCTACATCTGCAACGTGA
ATCACAAGCCCAGCAACACCAAGGTGGACAAGAAAGTTGAGCCCAAATCTTGTGACAAAACTCACACATGCCCACCG
TGCCCAGCACCTGAACTCCTGGGGGGACCGTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGTTCAACTGGTACGTGG
ACGGCGTGGAGGTGCATAATGCCAAGACAAAGCCGCGGGAGGAGCAGTACAACAGCACGTACCGTGTGGTCAGCGTC
CTCACCGTCCTGCACCAGGACTGGCTGAATGGCAAGGAGTACAAGTGCAAGGTCTCCAACAAAGCCCTCCCAGCCCC
CATCGAGAAAACCATCTCCAAAGCCAAAGGGCAGCCCCGAGAACCACAGGTGTACACCCTGCCCCCATCCCGGGATG
AGCTGACCAAGAACCAGGTCAGCCTGACCTGCCTGGTCAAAGGCTTCTATCCCAGCGACATCGCCGTGGAGTGGGAG
AGCAATGGGCAGCCGGAGAACAACTACAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCATGCTCCGTGATGCATGAGGCTCTGCACA
ACCACTACACGCAGAAGAGCCTCTCCCTGTCTCCGGGTAAATGA (SEQ ID NO: 16)


DEVQLVESGGGLIQPGGSLRLSCAASGLTVSSNYMSWVRQAPGKGLEWVSLIYPGGSTYYADSVKGRFTISRDNSNN
TLYLRMNSLRAEDTAVYYCARDLPHIAATGRVWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK (SEQ ID NO: 17)




2) EV053286M 109W (Light chain)

GACGACATCCAGATGACCCAGTCTCCATCCTCCCTGTCTGCATCTGTAGGAGACAGAGTCGCCATCACTTGCCAGGC
GAGTCAGGACATTAACAAGTATTTAAATTGGTATCAGCAGAAACCAGGGAAAGCCCCTAAGCTCCTGATCTACGATG
CATCCAATTTGGAAACAGGGGTCCCATCAAGGTTCAGTGGAAGTGGATCTGGGACAGATTTTACTTTCACCATCAGC
AGCCTGCAGCCTGAAGATTTTGCAACATATTACTGTCACCAGTATGATAATCTCCCTCCGACTTTCGGCCCTGGGAC
CAAAGTGGATATCAAACGAACTGTGGCTGCACCATCTGTCTTCATCTTCCCGCCATCTGATGAGCAGTTGAAATCTG
GAACTGCCTCTGTTGTGTGCCTGCTGAATAACTTCTATCCCAGAGAGGCCAAAGTACAGTGGAAGGTGGATAACGCC
CTCCAATCGGGTAACTCCCAGGAGAGTGTCACAGAGCAGGACAGCAAGGACAGCACCTACAGCCTCAGCAGCACCCT
GACGCTGAGCAAAGCAGACTACGAGAAACACAAAGTCTACGCCTGCGAAGTCACCCATCAGGGCCTGAGCTCGCCCG
TCACAAAGAGCTTCAACAGGGGAGAGTGTTAG (SEQ ID NO: 18)


DDIQMTQSPSSLSASVGDRVAITCQASQDINKYLNWYQQKPGKAPKLLIYDASNLETGVPSRFSGSGSGTDFTFTIS
SLQPEDFATYYCHQYDNLPPTFGPGTKVDIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC (SEQ ID NO: 19)

[FIG. 4]

[FIG. 5]

| RBD | Ka (1/Ms) | Kd (1/s) | KD (M) |
|---|---|---|---|
| WILD STRAIN | $1.64 \times 10^6$ | $6.29 \times 10^{-4}$ | $3.84 \times 10^{-10}$ |
| ALPHA STRAIN | $1.30 \times 10^6$ | $5.87 \times 10^{-4}$ | $4.51 \times 10^{-10}$ |
| BETA STRAIN | $2.30 \times 10^6$ | $2.60 \times 10^{-3}$ | $1.13 \times 10^{-09}$ |
| GAMMA STRAIN | $1.00 \times 10^6$ | $7.73 \times 10^{-4}$ | $7.71 \times 10^{-10}$ |
| DELTA STRAIN | $1.28 \times 10^6$ | $4.86 \times 10^{-4}$ | $3.81 \times 10^{-10}$ |
| KAPPA STRAIN | $1.87 \times 10^6$ | $7.91 \times 10^{-4}$ | $4.24 \times 10^{-10}$ |
| OMICRON BA.1 STRAIN | $4.66 \times 10^5$ | $1.50 \times 10^{-2}$ | $3.22 \times 10^{-08}$ |

[FIG. 6]

**286 109S**

| WILD STRAIN | ALPHA STRAIN | BETA STRAIN |
|---|---|---|
| **IC50: 84.5 ng/mL** | **20.8 ng/mL** | **60.3 ng/mL** |

ANTIBODY CONCENTRATION (ng/mL)

**286 109S**

| GAMMA STRAIN | DELTA STRAIN | KAPPA STRAIN |
|---|---|---|
| **IC50: 3.7 ng/mL** | **41.6 ng/mL** | **26.3 ng/mL** |

ANTIBODY CONCENTRATION (ng/mL)

| **286 109S** | **286 109W** |
|---|---|
| OMICRON BA.1 STRAIN | OMICRON BA.1 STRAIN |
| **IC50: 2.97 µg/mL** | **2.05 µg/mL** |

ANTIBODY CONCENTRATION (ng/mL)

● Raw data ——— Curve fitting      E6/TMPRS2, MOI=0.01

[FIG. 7]

OMICRON BA.2 STRAIN

EV053286 109S      Reg10933+Reg10987

IC50:    44.5 ng/mL      1194.6 ng/mL

ANTIBODY CONCENTRATION (ng/mL)

● Raw data   ——— Curve fitting

E6/TMPRS2, MOI=0.01

[FIG. 8]

[FIG. 9]

[FIG. 10]

1. LY-CoV1404 30mg/kg
2. EV053286M109W 30mg/kg
3. EV053286M109W 10mg/kg
4. NEGATIVE CONTROL ANTIBODY 30 mg/kg

# EP 4 471 147 A1

**INTERNATIONAL SEARCH REPORT**

| International application No. |
|---|
| **PCT/JP2023/002584** |

| A. | CLASSIFICATION OF SUBJECT MATTER |
|---|---|

*C12N 15/13*(2006.01)i; *A61K 39/395*(2006.01)i; *A61P 11/00*(2006.01)i; *A61P 31/14*(2006.01)i; *C07K 16/10*(2006.01)i; *C12N 1/15*(2006.01)i; *C12N 1/19*(2006.01)i; *C12N 1/21*(2006.01)i; *C12N 5/10*(2006.01)i; *C12N 15/63*(2006.01)i; *C12P 21/02*(2006.01)i; *C12P 21/08*(2006.01)i
FI: C12N15/13 ZNA; C12N15/63 Z; C07K16/10; C12P21/08; C12N1/19; C12N1/21; C12N1/15; C12N5/10; A61K39/395 S; A61P31/14; C12P21/02 C; A61P11/00

According to International Patent Classification (IPC) or to both national classification and IPC

| B. | FIELDS SEARCHED |
|---|---|

Minimum documentation searched (classification system followed by classification symbols)

C12N15/13; A61K39/395; A61P11/00; A61P31/14; C07K16/10; C12N1/15; C12N1/19; C12N1/21; C12N5/10; C12N15/63; C12P21/02; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN), JSTPlus/JMEDPlus/JST7580 (JDreamIII), UniProt/GeneSeq, Japio-GPG/FX , PubMed

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WANG, L. et al. Ultrapotent antibodies against diverse and highly transmissible SARS-CoV-2 variants. Science. 2021, vol. 373, no. 6556, abh1766, pp. 1-14 fig. 2, abstract | 1, 5-16 |
| Y | fig. 2, abstract | 1-16 |

| ✓ Further documents are listed in the continuation of Box C. | ✓ See patent family annex. |
|---|---|

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2023** | **18 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

39

EP 4 471 147 A1

**INTERNATIONAL SEARCH REPORT**

| International application No. |
| --- |
| **PCT/JP2023/002584** |

**C.    DOCUMENTS CONSIDERED TO BE RELEVANT**

| Category* | Citation of document, with indication, where appropriate, of the relevant passages | Relevant to claim No. |
| --- | --- | --- |
| X | News Release, 富山大学独自のコア技術を結集し、新型コロナウイルスの多種の変異株感染を防御できるヒト・スーパー中和抗体を新規に取得, [retrieved on 06 April 2023], 国立大学法人 富山大学総務部総務課広報・基金室, 16 June 2021, retrieved from the Internet, https://www.u-toyama.ac.jp/wp/wp-content/uploads/20210616.pdf, non-official translation (New acquisition of human super-neutralizing antibodies that can protect against infection with various mutant strains of the new coronavirus by bringing together the unique core technologies of the Univ. of Toyama. General Affairs Division, General Affairs Department, UNIV. OF TOYAMA NATIONAL UNIVERSITY CORP.) page 1, first paragraph, page 2, second paragraph, fig. 1 | 1, 5-16 |
| Y | page 1, first paragraph, page 2, second paragraph, fig. 1 | 1-16 |
| X | WO 2021/045836 A1 (REGENERON PHARMACEUTICALS, INC.) 11 March 2021 (2021-03-11) claims 1-80, example 12 | 1, 5-16 |
| Y | claims 1-80, example 12 | 1-16 |

Form PCT/ISA/210 (second sheet) (January 2015)

**INTERNATIONAL SEARCH REPORT**

| International application No. |
| --- |
| **PCT/JP2023/002584** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.

**PCT/JP2023/002584**

| Patent document cited in search report | | | Publication date (day/month/year) | Patent family member(s) | | | Publication date (day/month/year) |
|---|---|---|---|---|---|---|---|
| WO | 2021/045836 | A1 | 11 March 2021 | US | 10787501 | B1 | |
| | | | | EP | 3889177 | A1 | |
| | | | | JP | 2022-536429 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021045836 A1
- US 11447541 B1
- WO 2021045836 A
- WO 2007039682 A
- WO 2007100083 A
- WO 2007011041 A
- WO 2011091078 A
- WO 2006071877 A

### Non-patent literature cited in the description

- **WU F. et al.** *Nature*, vol. 579 (7798), 265-269
- **ZHOU P. et al.** *Nature*, March 2020, vol. 579 (7798), 270-273
- **HOFFMANN M. et al.** *Cell*, 16 April 2020, vol. 181 (2), 271-280. e8
- **WALLS AC et al.** *Cell*, 16 April 2020, vol. 181 (2), 281-292. e6
- **WU Y. et al.** *Science*, 12 June 2020, vol. 368 (6496), 1274-1278
- **ZHOU P. et al.** *Nature*, 2020, vol. 579, 270-273
- **WARD ES et al.** *Nature*, 1989, vol. 341, 544-546
- **RIECHMANN L. et al.** *Nature*, 1988, vol. 332, 323-327
- **KARLIN S ; ALTSCHUL SF**. *PNAS*, 1990, vol. 87, 2264-2268
- *PNAS*, 1993, vol. 90, 5873-5877
- **ALTSCHUL SF et al.** *J Mol Biol*, 1990, vol. 215, 403-410
- **J. SAMBROOK et al.** Molecular Cloning, A Laboratory Manual. Cold Spring Harbor Laboratory Press, 1989
- **BOULIANNE GL et al.** *Nature*, 1984, vol. 312, 643-646
- **JONES PT et al.** *Nature*, 1986, vol. 321, 522-525
- **OMASA T.** *J. Biosci. Bioeng.*, 2002, vol. 94, 600-605
- **NIWA R et al.** *Clin. Cancer Res.*, 2004, vol. 10, 6248-6255
- **KANDA Y. et al.** *Glycobiology*, 2007, vol. 17, 104-118
- **KOZBOR D. ; RODER JC**. *Immunol Today*, 1983, vol. 4, 72-9
- **MATSUYAMA S et al.** *PNAS*, 2020, vol. 117, 7001-7003
- **URANO E et al.** *PNAS*, 2021, vol. 118 (43), e2104847118
- **COPIN R et al.** *Cell*, 2021, vol. 184, 3949-3961